# EUROPEAN PATENT APPLICATION

(11) **EP 4 389 192 A1**
(43) Date of publication of application: **26.06.2024**
(21) Application number: 22461650.8
(22) Date of filing: 23.12.2022
(51) Int. Cl.: A61N 1/04, A61N 1/36

(54) **NEURAL STIMULATION SYSTEM FOR SUPPORTING SPEECH AND LANGUAGE SKILLS OF A USER, ESPECIALLY OF A USER HAVING APHASIA**

(71) Applicant: Neuro Device Group S.A., 01-510 Warsaw (PL)
(72) Inventor: Malej, Krzysztof Mateusz, 01-510 Warsaw (PL); Garnier, Justyna Julia, 01-510 Warsaw (PL); Kinasiewicz, Joanna, 01-510 Warsaw (PL); Szostakowska, Sylwia, 01-510 Warsaw (PL); Orzechowski, Mateusz Marek, 01-510 Warsaw (PL)
(74) Representative: Schmidt, Christian

(57) **Abstract**

The disclosure relates to a neural stimulation system (100) for supporting speech and/or language skills of a user (120), comprising:
- an electrical stimulation unit (202) that is configured to generate an electrical stimulation signal for the brain (130) of the user (120),
wherein the neural stimulation system (100) is configured to support the user (120) by stimulating areas of the brain (130) of the user (120) that are involved in speech and/or language processing and by using stimulation parameters to generate the electrical stimulation signal that are appropriate to enhance the word processing of the user (120).

## Description

The disclosure relates to a neural stimulation system for supporting speech and/ language skills, e.g. of a user suffering from aphasia. Furthermore, related items will be described.

### Summary

It is an object of the disclosure to provide a neural stimulation system for supporting speech and/or language skills of a user. Preferably, the neural stimulation system shall enable fast improvement of the speech and/or language skills of the user. More preferably, the neural stimulation system shall be simple and/or shall have a simple user interface. Especially, corresponding usage of such a neural stimulation system, corresponding methods and corresponding items shall be provided.

This object may be achieved by the neural stimulation system according to claim 1. Corresponding usage, method and other items are claimed in the independent claims. Further embodiments are provided in the dependent claims.

In one aspect a neural stimulation system for supporting speech and/or language skills of a user is provided, comprising:
- An electrical stimulation unit that is configured to generate an electrical stimulation signal for the brain of the user.

According to an embodiment, the neural stimulation system may be configured to support the user:
- by stimulating areas of the brain of the user that are involved in speech and/or language processing, and/or
- by using stimulation parameters to generate the electrical stimulation signal that may be appropriate to enhance the word processing of the user.

The system may be implemented as an apparatus comprising several units.

The stimulation parameters may comprise at least one of the following:
- Current (max. and/or min value) symmetrical or asymmetrical with regard to zero ampere,
- Frequency,
- Voltage,
- Duration,
- Pause length (constant pauses, etc.).

The neural stimulation system may also be named as a brain stimulation system as the brain is a part of the nervous system that is a stimulation target in disclosed embodiments.

The neural stimulation system may allow to perform a stimulation in real time and/or synchronously with real life activities of the user, e.g. without time delays greater than 1 second or greater than 30 seconds. Alternatively, the simulation may be preparation stimulation, i.e. stimulation not during planned activity involving language processing, e.g. hearing, speaking, etc. The preparation stimulation may be performed more than 5 minutes or more than 10 minutes before the real life activity of the user that comprises speech and/or language processing by the user/patient.

The neural stimulation system may allow to perform a stimulation in response to:
- A demand of the user, and/or
- Depending on time slots planned by the user in advance, and/or
- Depending on a state of the user that is monitored, whereby the stimulation may be started when the state indicates that the arousal and/or engagement of the user, e.g. in speech and/or language processing in a real life activity increases and/or when the cognitive load (workload) increases, e.g. due to enhanced speech and/or language processing. Preferably, the stimulation may be started only after prior enabling the stimulation feature by the user.

The language may be a first language or natural language of the user/patient, e.g. mother tongue language. Alternatively the language may be a second language, e.g. a languages learned in addition to the first language, or at least on further language.

The user may be a normal healthy person, e.g. a person which wants to improve their speech and/or language processing in the first language or in the second language.

The user may be a healthy older person with mitigated word and/or speech and/or language processing skills compared to at least one year ago, at least 10 years ago etc. The older person may be older than 60 years, older than 70 years or older than 80 years.

The user may be a patient, e.g. a patient having mitigated speech and/or language processing skills. The patient may suffer from an illness that mitigates word processing of the patient, e.g. aphasia.

The real life activity may be an activity in which the user/patient is independent of a language training system, e.g. outside a clinic environment. Thus, the real life activity may take place in a non-clinical environment, e.g. in a working environment (office, e.g. take part in a meeting or telephone conversation, video call, etc., factory, etc.), in a daily life activity, e.g. eating in a restaurant, meeting people meeting friends, etc.

The stimulation may comprise:
- An electrical stimulation signal, this may be prone for mobile stimulation devices,
- An electro-magnetic stimulation signal, and/or
- A magnetic stimulation signal.

According to an embodiment, the neural stimulation system may comprise a user interface that may be configured to receive input of the user, e.g. input that indicates that the user wants to start stimulation preferably immediately.

In this embodiment "On demand" should be understood as when the user intends to perform activities and wishes to use the neural stimulation device to increase performance, e.g. explicitly. In such a case, the user may take an action to enable the device which may start stimulation immediately and/or which may then be followed by pauses/stimulation phases of the given length.

Thus, the user may get the stimulation when he/she has the feeling that he/she needs support for their speech and/or language processing.

Consideration of a stimulation budget may be advantageous in case of explicit start of stimulation, e.g. in order to not mitigate health of the user or to not to impair the state of a patient further. Usage of smaller doses within the budget may increase positive effects on language processing and/or may spread the stimulation budged over an increased time frame.

According to an embodiment, the neural stimulation system may comprise a scheduler unit that may be configured to provide a planning functionality allowing the user to specify time slots in which he wants to have at least one stimulation. Preferably the scheduler unit may distribute stimulation depending on a stimulation budget to the specified time slots.

Consideration of a stimulation budget when distributing stimulation to the time slots may be advantageous, e.g. in order to not mitigate health of the user or to not to impair the state of a patient further. Usage of smaller doses within the time slots may increase positive effects on speech and/or language processing and/or may spread the stimulation budged over an increased time frame.

Thus, a control unit may comprise a stimulation planning functionality that may automatically distribute stimulation periods and pause periods over time predefined by a user or predefined as a reference timeframe, e.g. in order to optimize stimulation effects over total length of planned activity. The overall duration of the stimulation periods may form a budget for stimulation within the reference timeframe.

Improved speech and/or language processing may be used as an optimization criterion. However, a more specific criterion or more specific criteria may be used as well.

Stimulation may be started for the implicit demand case immediately if there is enough "budget" left.

According to an embodiment, the neural stimulation system may comprise a user condition monitoring unit configured to determine a state of the user. The neural stimulation system may be configured to start stimulation or to generate a request to the user in order to start stimulation if e.g. an increased arousal and/or engagement or e.g. an increased cognitive load is detected based on the determined state of the user.

Preferably the neural stimulation system may be configured to generate a request to the user whether they want to start the stimulation. Preferably the stimulation may be started if the user decides to start stimulation in an answer or confirmation to the request. Thus, the user may become aware that he or she is in a state that triggers implicit demand. Moreover, the user may decide whether to make use of the stimulation or not, e.g. depending on other criterion, e.g. if further activities are planned on the same day or not.

The usage of a monitoring function may be regarded as detecting an "implicit demand" of the user or patient for support stimulation during a daily activity, e.g. a real life activity involving speech and/or language processing.

Consideration of a stimulation budget may be advantageous if stimulation is started implicitly based on the state of the user, e.g. in order to not mitigate health of the user or to not to impair the state of a patient further. Usage of smaller doses may increase positive effects on speech and/or language processing and/or may spread the stimulation budged over an increased time frame.

According to an embodiment, stimulation may also be stopped "on implicit demand", e.g. if the user condition monitoring unit determines a decrease arousal and/or engagement during the daily life activity of the user, e.g. due to less speech and/or language processing. Again, the user may be asked whether he/she wants to stop stimulation or not, e.g. in order to raise awareness that he/she is again in a relaxed state or for other reasons (enhanced planning, etc.)

Stimulation may be started for the implicit demand case immediately if there is enough "budget" left.

However, combinations of all three modes may also be possible, e.g. a) on explicit demand, b) on implicit demand c) within planned time slots. Thus, mode a) may be combined with mode c), e.g. by allowing explicit demand only within the planned time slots or alternatively also outside of the planned timeslots, followed by redistribution of the lower simulation budget. As a further example, mode b) may be combined with mode c), e.g. by allowing implicit demand only within the planned time slots or alternatively also outside of the planned timeslots, followed by redistribution of the lower simulation budget.

According to an embodiment, the neural stimulation system may comprise a user condition monitoring unit configured to determine a state of the user. The neural stimulation system may be configured to stop stimulation if an decreased arousal and/or engagement or an decreased cognitive load is detected based on the determined state of the user. Preferably, the neural stimulation system may be configured to generate a request to the user whether they want to stop the stimulation. Preferably, the stimulation may be stopped if the user decides to stop the stimulation in answer to the request. Thus, a the stimulation is only performed if it is really necessary. This may allow to use a stimulation budget effectively, e.g. to reduce the budget only if it is necessary.

According to an embodiment, alternatively or additionally to the embodiment of the previous paragraph, the neural stimulation system may be configured to stop stimulation or to generate a request to the user in order to stop stimulation if a condition or state of the user is indicative of a pathological state of the user. Thus, interference or neural stimulation and of pathological conditions/ states of the user/patient may be effectively prevented, e.g. in order to protect health of the user/patient. An example for a pathological state may be a heart disease or a heart defect. Again, the neural stimulation system may comprise a user condition monitoring unit configured to determine a state of the user.

According to an embodiment, the neural stimulation system may be configured to support the user by stimulating areas of the brain of the user that are involved in speech and/or language processing and/or by using stimulation parameters to generate the electrical stimulation signal that are appropriate to enhance the word processing of the user at the same time at which the stimulation is made.

"At the same time" may mean that the support (positive effects of stimulation) and the stimulation take place simultaneously, e.g. within a time period of less than 10 seconds, less than 5 seconds or even of less than 1 second. To give only an example, a lower limit may be set by characteristics of the brain, e.g. processing speed of brain, or a neuron group and or of a neuron, especially of the corresponding oscillation cycle.

Thus, the user/patient may be able to profit immediately from the support stimulation, e.g. at the "moment" at which the stimulation is made.

According to an embodiment of the neural stimulation system according to any one of the embodiments mentioned above, the user may be a patient suffering from an illness that has mitigated his/her speech and/or language processing skills.

Thus, the user/patient may suffer from a speech and/or language disorder, e.g. from aphasia. A pilot study of the applicant proofed immediately improvement of the speech and/or language processing skills for a particular speech and/or language processing task and a particular stimulation current frequency, see further detail given below. Based on knowledge of neuroscience, improvement for other speech and/or language processing tasks and/or other frequencies may be expected as well.

The electrical stimulation unit may be configured to generate a transcranial alternating current stimulation (tACS), preferably using an alternating current frequency in the range of 60 Hz to 80 Hz or 70 Hz to 80 Hz, 60 Hz to 100 Hz or 70 Hz to 100 Hz, 60 Hz to 150 Hz or 70 Hz to 150 Hz; 60 Hz to 250 Hz or 70 Hz to 250 Hz, preferably at approximate 75 Hz or at exactly 75 Hz.

Frequencies within these ranges may improve speech and/or language processing skills, e.g. in a situation in which the user is autonomous in the manner in which he/she participates in the conversation with at least one other person in order to fulfill its function in daily life activity, e.g. attending a meeting, making a telephone conversation, participating in a video calli conference, buying a product, visiting a restaurant, etc.

Not only tACS (transcranial alternating current stimulation) may be used. Exemplarily, according to a further embodiment, tDCS (transcranial direct current stimulation) may also be used on implicit demand, on explicit demand and/or according to a scheduler for improving speech and/or language skills.

According to a further embodiment, tRNS (transcranial random noise stimulation) may be use, e.g. with a spectrum of the random frequencies within one of the ranges specified within this document in order to provide speech and/or language processing support in real time.

According to a further embodiment, tVCS (transcranial variable current stimulation) may be used, e.g. within one of the ranges specified within this document in order to provide speech and/or language processing support in real time. The frequency may be increased and/or decreased within the borders defined by the specific range.

According to an embodiment, the neural stimulation system may comprise a control unit that may be configured to control the electrical stimulation unit depending on the stimulation parameters. The control unit or another unit of the neural stimulation system may be configured to provide the electrical stimulation signal on demand, e.g. when the user intends to perform activities requiring intensive speech and language processing and/or when the user proactively enables and/or triggers brain stimulation device in order to improve speech and/or language performance.

Thus, stimulation may be performed as a direct and immediately answer of the neural stimulation system to the request, e.g. explicit request, of the user for support stimulation. Alternatively, the stimulation may be enabled as a direct and immediately answer of the neural stimulation system to the request, e.g. explicit request, of the user for support stimulation.

According to an embodiment, the control unit or a control unit or another unit of the neural stimulation system may be configured to dose the electrical stimulation signal in short intervals during the performed activity, e.g. the neural stimulation system may be configured to provide stimulation for a specific period of time, e.g. 10 minutes, and preferably to provide then a pause in the stimulation, preferably for a specific period of time in the range of 2 minutes to 15 minutes or in the range of 6 minutes to 12 minutes. These stimulation ranges may result in improved speech and/or language processing skills in real time.

Alternatively and/or additionally the control unit may be configured to control a pause length. The pause length may be preferably a specific period of time, e.g. a constant period of time, and/or a period of time in the range of 1 minute to 5 minutes or in the range of 1 minute to 3 minutes, e.g. 2 minutes. These pause ranges may result in improved speech and/or language processing skills in real time, e.g. especially in combination with the stimulation ranges mentioned above, e.g. in the preceding paragraph.

According to an embodiment, the control unit or a control unit may be configured to allow the user to adjust the pause length, preferably in order to distribute stimulation periods evenly over an anticipated duration of activity of the user. Thus, the user may adjust the pause depending on their personal needs.

According to an embodiment, the control unit or a control unit may be configured to control the duration (budget) of stimulation in order not to exceed a maximum allowed total duration during a reference timeframe, wherein the reference timeframe may be e.g. a 24 hours timeframe or any other appropriate timeframe with correspondingly adapted duration of stimulation or budget for stimulation, e.g. doubling of the value for 48 hours or half of the value for 12 hours. Consideration of the budget may be advantageous with regard to safety of the patient and/or with regard optimal effects for improvement of speech and/or language processing skills of the user/patient and/or with regard to other reasons.

The duration of stimulation (budget) may be in the range of 30 minutes to 120 minutes or in the range of 45 minutes to 90 minutes, e.g. 60 minutes per 24 hours or per other appropriate timeframe with correspondingly adapted duration. According to the pilot study made by the applicant, this range may provide good improvements of speech and/or language processing skills during real time stimulation in a training situation. It is expected that the same range may provide similar good results in everyday situations, e.g. in real life situation outside of or out of a training of the user/patient, especially of a training involving tasks that have been prepared before the training is started such as picture naming tasks, etc.

A total duration (budget) per timeframe may be defined as the sum of all stimulation periods/phases performed during the reference time frame, e.g. during the 24 hours timeframe. The other time of the reference frame may be filled by short pauses between stimulation phases and by time intervals in which not stimulation is performed at all.

More than 60 minutes per 24 hours overall budget may also be used for stimulation. The upper limit may be set by prevention of negative effects to the user or to the patient and/or by having no decreasing of speech and/or language processing capacity of the user/patient.

The budget may be more than 30 minutes per 24 hours or a correspondingly scaled value for another appropriate timeframe, e.g. the lower limit may depend on length of activity of user and the minimum value may not jeopardize overall improvement of speech and/or language skills.

According to an embodiment, the neural stimulation system may comprise at least one stimulation electrode (e.g. at least one first electrode) that is configured to be mounted on to the head of the user, preferably non-invasively. A dry electrode or a semi dry electrode may ease usage of the neural stimulation system in daily activities, e.g. because the user has not to apply a gel or has not so often apply a gel to the electrodes. However, a gel or other electrically conducting materials on the electrode(s) may have positive effects on the transmission of the stimulation current from the electrode to the cranium and thereafter to the region of the brain that has to be stimulated or in the other direction. Efficient transmission of the current may improve overall results of the stimulation, e.g. improve the speech and/or language processing skills of the user/patient.

The at least one stimulation electrode (e.g. first electrode) may be configured to be positioned on the left side of the head of the user, e.g. close to areas of the brain related to speech and/or language functions, especially for right handed users who have the main language processing areas usually within the left hemisphere of the brain. This does not exclude that areas in the other hemisphere may not play a role in speech and/or language processing and/or may be stimulated as well. However, studies may be performed first in order to find the appropriate stimulation parameters, brain areas, etc.

The neural stimulation system may comprise at least one second electrode (e.g. a stimulation electrode or a counter electrode) that may be configured to be positioned on the right side of the body of the user or on the right side of the head of the user.

Although a non-invasive arrangement of the electrodes is preferred, it at least one of the electrodes or all electrodes may be arranged invasively, e.g. subcutaneously.

According to an embodiment, the neural stimulation system may comprise a brain stimulation device, preferably comprising a wearable headband, e.g. a stretchable or resiliently deformable headband. The headband may allow to generate a force that presses the electrode(s) against the head of the user/patient in order to provide a good electrical connection between the electrodes and the skin on the cranium. Removal of hair in regions used for arrangement of the electrodes may also provide an electrical connection having low resistance or low impedance. However, alternatively or additionally other technical means may be used to arrange the electrode(s) securely or safe on the head of the user, e.g. a non-elastic headband. Thus, the non-elastic headband may have a length that is adjustable to the circumference of the head of the user/patient.

The at least one electrode may be attached to the brain stimulation device, e.g. mechanically to other units of the brain stimulation device and/or via electrical connections. The brain stimulation device may be configured such that the user can wear it for prolonged periods of time, e.g. for more than one week, more than one month, or even for more than one year. Thus, the brain stimulation device may be a robust device, that may be cleaned regularly. The brain stimulation device may be usable for less than 10 years to give only an example.

According to an embodiment, the neural stimulation system may comprise:
- A user condition monitoring unit or functionality for detecting a state of the user, e.g. mental, physical, etc., and/or
- A control unit or the control unit that may be configured to start the stimulation automatically depending on the detected state, e.g. without knowledge of the user or with prompting for a user confirmation for performing the stimulation without a previous request of the user to get a brain stimulation or neural stimulation, e.g. preferably if an increased arousal and/or engagement or an increased cognitive load is detected based on the determined state of the user. Alternatively or additionally, a control unit or the control unit may be configured to generate a automatically request to the user in order to start stimulation depending on the detected state, e.g. preferably if an increased arousal and/or engagement or an increased cognitive load is detected based on the determined state of the user, e.g. in order to raise awareness of the user that stimulation takes place and/or to allow spare efficient usage of a stimulation budget.

Thus, the user may concentrate on his daily activities without spending too much effort to choose the moment to start the brain stimulation or neural stimulation.

The user condition monitoring functionality may be configured to determine the current condition experienced by the user, e.g. based on user condition data indicative for the current condition of the user and/or based on one or more physiological parameters of the user. The one or more physiological parameter(s) of the user may be monitored by the brain stimulation system during performing the speech and language related activities by the user.

At least one of the user condition data and/or the one or more physiological parameter may be used to trigger the start of the stimulation when the user experiences excessive arousal and/or engagement level or cognitive load in order to support the user in the performed activity. Thus, an easy way to start stimulation automatically or to ask automatically for user confirmation to start stimulation may be provided.

The user condition data may be data indicative for the current arousal and/or engagement level and/or cognitive load of the user, e.g. fatigue and/or mental load. The one or more physiological parameter of the user may be based on electrodermal activity and/or on the pulse frequency and/or heart rate and/or blood pressure of the user and/or on heart rate variability of the heart of the user and/or level of blood oxygenation. These physiological parameter(s) may be comparably easily to be detected.

According to an embodiment, the neural stimulation system may comprise:
- a user condition monitoring unit for detecting a state of the user, e.g. mental, physical, etc., and
- a control unit that is configured to stop the stimulation automatically depending on the detected state, e.g. without knowledge of the user, or to generate automatically a request to the user in order to stop the stimulation depending on the detected state, preferably if a decreased arousal and/or engagement or an decreased cognitive load is detected based on the determined state of the user or if a condition or state of the user is indicative of a pathological state of the user.

The technical effects and/or medical effects may be as mentioned already above, e.g. to prevent overstimulation and/or to use a stimulation budget effectively and/or efficiently, etc.

The user condition monitoring functionality may be configured to determine user condition data indicative for the current condition of the user experienced by the user and/or one or more physiological parameters of the user which may be monitored by the brain stimulation system during performing the speech and language related activities by the user.

At least one of the user condition data and/or the one or more physiological parameters may be used to trigger the stop of the stimulation when the user experiences decreased arousal and/or engagement level or cognitive load in order to end support of the user it he does not need the support anymore in the performed activity.

The user condition data may be data indicative for the current arousal and/or engagement level and/or cognitive load of the user, e.g. fatigue and/or mental load.

Preferably, the one or more physiological parameters of the user may be based on electrodermal activity and/or on the pulse frequency and/or heart rate and/or blood pressure of the user and/or on heart rate variability of the heart of the user and/or level of blood oxygenation.

According to an embodiment, the neural stimulation system may be configured to be portable by the user, e.g. on the body of the user, especially using a waist belt. The neural stimulation system may have a weight of less than 1 kg (kilogram) or of less than 500 g (gram). To give only an example for a lower limit, the neural stimulation system may have a weight of more than 100 g. The neural stimulation system may comprise an accumulator, e.g. a rechargeable battery, or a non-rechargeable battery, e.g. in order to enable a mobile device that is not connected to the grid for power supply during usage, especially during stimulation of the brain of the user/patient. The weights mentioned above may include the weight of the battery. Lower weight may improve user comfort. Other components of an neural stimulation arrangement comprising the neural stimulation system may also be portable and/or have a low weight, e.g. neural (brain) stimulation device, e.g. wearable on the head, and/or user condition monitoring device, e.g. wearable on the wrist of the user/patient, etc. The mentioned weights may include all components of the neural stimulation arrangement according to another further embodiment.

The greatest extension of the neural stimulation system may be less than 30 cm (centimeters), less than 20 cm, less than 15 cm or even less than 10 cm. The greatest lateral extension may be more than 5 cm to give only an example.

The neural stimulation system may comprise a user interface that may be configured to allow input of the user and/or to output control information to the user. Input may relate to:
- Setting of selected stimulation parameters, e.g. length of pause, preferably within specific limits, and/or
- Optionally, confirming, e.g. by the user/patient, that stimulation shall be started, e.g. if user condition indicates increased arousal and/or engagement and/or cognitive load, and/or
- Optionally, confirming, e.g. by the user/patient, that stimulation shall be ended, e.g. if user condition indicates decreased arousal and/or engagement and/or cognitive load.

The user interface may comprise a touchscreen and/or at least one button or switch.

A telemetry or transmission function or a local input interface may be used to set stimulation parameters by practitioner or physician. The local input interface may be only activated after checking an ID (Identifier), password or other data for authentication, e.g. in order to provide appropriate setting of other stimulation parameters, e.g. of parameters that are not allowed to be set by the user or patient themselves.

The output of the user interface may relate to:
- Stimulation parameters as mentioned above, e.g. using display (LCD, liquid crystal display), e.g. for checking of the current value(s) by the user or patient or other person (practitioner, physician, etc.), and/or
- Signal whether stimulation is active or not, e.g. using visual signal, (LED, light emitting diode),
- Optionally, requesting the user/patient whether stimulation shall be started, e.g. if user condition indicates increased arousal and/or engagement and/or cognitive load, and/or
- Optionally, requesting the user/patient whether stimulation shall be ended, e.g. if user condition indicates decreased arousal and/or engagement and/or cognitive load.

According to another aspect, usage or use of the neural stimulation system according to any one of the embodiments mentioned above within specific situations is provided. Thereby, the word processing of the user/patient may comprise hearing of words spoken by at least one other person and/or understanding the words spoken by the at least one other person and/or speaking words to the at least one other person. The neural stimulation system may be used to support the user in a working environment of the user. The working environment is a central part of the social life of the user/patient. Support in this central part may have a synergistic effect for rehabilitation and/or recovery, e.g. for reestablishing full or at least sufficient speech and/or language processing skills of aphasia patients.

Alternatively or additionally, the neural stimulation system may be used to support the user in another environment in which the user has to communicate to the at least one other person with enhanced speech and/or language skills outside of a language training situation. Thus, the system may be used in everyday situation, enabling the user/patient to be autonomous and/or to mitigate the impact of a speech disorder to his life.

According to a further aspect, a method for supporting speech and/or language performance of a user is provided. The method may comprise:
- Providing a neural stimulation system that is configured to generate an electrical stimulation signal for the brain of the user, e.g. a neural stimulation system according to one of the embodiments mentioned above.

The neural stimulation system may be configured to support the user by stimulating areas of the brain of the user that are involved in speech and/or language processing and/or by using stimulation parameters to generate the electrical stimulation signal that are appropriate to enhance the word processing of the user preferably at the same time at which the stimulation is made in these areas.

The word processing of the user may again comprise hearing of words spoken by at least one other person and understanding the words spoken by the at least one other person and speaking words to the at least one other person.

According to an embodiment, the method may comprise further:
- Supporting the user by the neural stimulation system in a working environment of the user or in another environment in which the user has to communicate to the at least one other person with enhanced speech and/or language skills outside of a speech and/or language training situation.

Thus, the technical and medical effects mentioned above may be valid. Moreover, the method may allow to implement the "on demand" features mentioned above and claimed in claims 2 (explicit by user), 3 (scheduler) and/or 4 (state of user monitoring, implicit) of this application.

tACS, tDCS or other appropriate brain or neural stimulations may be used for the method as well.

The method may be performed using a neural (brain) stimulation system according to one of the embodiments mentioned above. Thus, the technical effects mentioned for the neural (brain) stimulation system are also valid for the method and vice versa.

According to a further embodiment, the method may be used for supporting speech and/or language performance of a user/patient, preferably of a patient suffering from speech and language disorders, e.g. aphasia. The method may comprise:
- Providing external transcranial alternating current stimulation (tACS) to at least one speech and/or language-related area of a brain of a user, especially a patient, wherein the frequency of a current of the tACS is a one frequency selected from within the high gamma frequency range of 60 Hz to 250 Hz or 70 Hz to 250 Hz. These ranges are supported by a pilot study of the applicant, directly or indirectly using knowledge of neuroscience.

The stimulation may be provided on demand of the user/patient, e.g., when a user intends to perform activities requiring intensive speech and language processing. The one frequency of the current of the tACS may be within a frequency range of 60 Hz to 80 Hz or 70 Hz to 80 Hz, preferably at approximate 75 Hz. Thus, only one frequency may be used for stimulation enabling, e.g. optimal support of the language processing of the user.

According to a further embodiment of the method, the electrical stimulation signal, e.g. a transcranial alternating current stimulation, may be dosed in short intervals during the performed activity. The neural stimulation system may provide stimulation for a specific period of time, e.g. 10 minutes, and then provides a pause in the stimulation. The stimulation or a request to the user to start stimulation may be triggered automatically. The pause may be automatically as well. Thus, the technical effects and/or the medical effects mentioned above do apply, e.g. higher user comfort and optimal stimulation and/or optimal distribution of a stimulation budget within a reference time frame, etc.

According to a further embodiment of the method, the pause length may have a specific length, preferably in the range of 1 minute to 5 minutes or in the range of 1 minute to 3 minutes, e.g. 2 minutes. This range may allow to avoid overstimulation or overtraining of the brain and/or neurons and/or neuron groups of the user/patient that are stimulated to oscillate and/or that are stimulated otherwise by the stimulation current. On the other side, the short break may prevent that the speech and/or language processing performance of the user/patient decreases or decreases too much.

According to a further embodiment of the method, the pause length may be adjusted by the user in order to distribute stimulation periods evenly over the anticipated duration of activity. Thus, the user may select a pause length that he/she experiences as agreeable or useful, e.g. within pre-given limits for safety or other reasons as mentioned above.

The time for the stimulation and the time for the pause may form a specific time interval for one stimulation phase including the pause. Thus, making the pause longer may shorten the stimulation duration during one stimulation phase and vice versa. Shortening of the duration of stimulation during one phase may allow to distribute the overall stimulation budget, e.g. for one day (24 hours) to a longer period of time appropriate for the anticipated duration of the activity of the user.

Alternatively or additionally, the simulation duration for a stimulation phase may be adjusted or may be adjustable by the user/patient or by another person, wherein the time for the stimulation and the time for the pause form a specific time interval for one stimulation phase including the pause, e.g. 10 minutes.

According to a further embodiment of the method according to any one of the embodiments mentioned above, the duration of stimulation may be controlled in order to not exceed a maximum allowed total duration during a reference timeframe, e.g. the 24 hours timeframe, e.g. 60 minutes per 24 hours or more than 60 minutes per 24 hours or less than 60 minutes per 24 hours, wherein a total duration is defined as sum of all stimulation periods performed during the reference time frame, e.g. the 24 hours timeframe. Other timeframes may be used as well, e.g. with the length of the stimulation adapted proportional to the change of the time frame compared to the 24 hour time frame. Technical effects and medical effects of these ranges for stimulation are mentioned above for corresponding features of the system.

According to a further embodiment of the method according to any one of the embodiments of the method mentioned above, especially according to the embodiment mentioned in the preceding paragraph, a stimulation planning functionality may automatically distribute stimulation periods and pause periods over a time, e.g. comprising at least one time slot, predefined by a user, preferably in order to optimize stimulation effects over total length of planned activity. The overall time, e.g. the sum of the time of the time slots, may be greater than the allowed stimulation (budget) within a reference time frame and/or also greater than the overall time specified by the user/patient, e.g. greater than the budget multiplied by factor 2 or even greater than the budget multiplied by factor 3.

The optimization during the distribution of the budget into the time slot(s) defined by the user/patient may relate to reaching improved speech and/or language performance of the user.

According to a further embodiment of the method according to any one of the embodiments of the method mentioned above, at least one stimulation electrode may be mounted on to the head of the user non-invasively. The at least one stimulation electrode may be positioned on the left side of the head of the user close to areas of the brain related to speech and language functions, especially for right handed users who have the main language processing areas usually on the left side of the head, i.e. within the left hemisphere of their brain. Thus, an optimal arrangement for most of the users/patients is provided.

According to a further embodiment of the method according to any one of the embodiments of the method mentioned above, especially of the embodiment mentioned in the preceding paragraph, at least one second stimulation electrode or at least on second electrode (counter electrode) is positioned on the right side of the body of the user or on the right side of the head of the user. The second electrode may be a stimulation electrode if arranged on the opposite side of the head compared to the side on which the first electrode is arranged, especially if an alternating current is used for stimulation. The second electrode may be a mere counter electrode if arranged e.g. on the right arm of the user or patient.

According to a further embodiment of the method according to any one of the embodiments of the method mentioned above, especially of the embodiment mentioned in the preceding paragraph, the at least one electrode may be attached to a brain stimulation device, preferably comprising a wearable headband, which the user may e.g. wear for prolonged periods of time. Technical effects and/or medical effects are mentioned above for the neural stimulation system.

According to a further embodiment of the method, an operation control system may comprise a user condition monitoring functionality which may be configured to determine user condition data indicative for the current condition of the user and/or one or more physiological parameters of the user which may be preferably monitored by the brain stimulation system during the performing of the speech and language related activities by the user.

The user condition data and/or the one or more physiological parameters of the user may be used to automatically trigger the start of the stimulation or to automatically trigger a request to the user to start stimulation, when the user experiences excessive cognitive load, preferably in order to support the user in the performed activity.

The user condition data may be data indicative for the current arousal and/or engagement and/or cognitive load of the user, e.g. fatigue and/or mental (e.g. cognitive) load (workload).

The one or more physiological parameter(s) of the user may be based on electrodermal activity and/or on the pulse frequency and/or heart rate and/or blood pressure of the user and/or on heart rate variability of the heart of the user. Blood oxygenation level may be considered as well.

Some of the abovementioned physiological parameters of the user, e.g. pulse frequency and/or heart rate and/or blood pressure and/or blood oxygenation level of the user may be indicative of pathological condition of the user, e.g. a heart disease, and may be used to automatically trigger the stop of the stimulation in order to support the safety of the user when the one or more of the parameters reach the critical value and/or to prevent interaction of neural stimulation with the disease.

According to a further aspect of the invention, a non-transitory computer readable medium, having stored therein instructions that are executable to cause a control unit to perform at least a part of or the method according to any one of the embodiments of the method mentioned above. Thus, the respective technical effects and/or medical effects of the method may apply the corresponding non-transitory computer readable medium as well. Examples of a non-transitory computer readable medium are a memory stick, e.g. an USB (universal serial bus) stick, an EEPROM (electrically erasable programmable read only memory), e.g. as part of an SSD (solid state disc), a magnetic storing disc (hard drive), CD (Compact disk) ROM, etc.

According to a further aspect of the invention, a computer program product is provided comprising machine readable instructions which when executed on a control unit cause a control unit to perform at least a part of or the method according to any one of the embodiments mentioned above. Thus, the respective technical effects and/or medical effects of the method may apply the corresponding computer program product. Examples of a computer program product are a program or program part transmitted via a data transmission network, e.g. via a data packet transmission network, especially via the Internet using the IP (Internet Protocol) protocol stack, see RFC's (Request for Comments of IETF (Internet engineering task force)), a program stored on a server in the internet, etc.

According to a further aspect of the invention, a system is provided, e.g. a computer system, comprising:
- At least one control unit, e.g. CPU (central processing unit) or MCU (micro controller unit), and
- A non-transitory computer readable medium, having stored therein instructions that are executable to cause a control unit to perform at least a part of or the method according to any one of the embodiments mentioned above.

Thus, the respective technical effects and/or medical effects of the method may apply the corresponding computer as well. The computer may be comprised within a smartphone, within a portable device, etc.

According to an aspect of the invention, a neural (brain) stimulation device is provided, comprising:
- A communication interface unit,
- At least one control unit connected to the communication interface,
- At least one electrode configured to be controlled by the control unit, and
- An electrical power unit configured to supply electrical power to the units of the neural stimulation device and to supply power to the at least one electrode in order to perform a brain stimulation of a brain of a user carrying the neural (brain) stimulation device.

The neural stimulation device may be wearable on the head of the user/patient. The neural stimulation device may be configured to be used as part of a neural stimulation arrangement that comprises also the neural stimulation system according to any one of the embodiments mentioned above. Thus, the same technical and/or medical effects that apply to the embodiments of the system may also apply to neural stimulation device.

The communication interface may be configured to communicate with a neural stimulation system, preferably with a neural stimulation system according to any one of the embodiments mentioned above, e.g. in order to receive and/or preferably also to confirm digital commands or digital parameters specifying details of an electrical stimulation of a brain of a user/patient who carries the neural stimulation device. A Bluetooth (may be a trademark, defined by the Bluetooth special interest group SIG) interface may be used, especially a low power interface, e.g. Bluetooth low energy (may be a trademark BLE). Other protocols may be used as well, e.g. ZigBee, etc.

The control unit may be configured to control a current and/or a voltage applied to the at least one electrode according to the digital commands and/or the digital parameters received via the communication interface. The control unit may comprise a CPU or a MCU.

The electrical power unit may comprise a rechargeable or non-rechargeable battery (accumulator) in order to enable mobile use of the neural stimulation device. However, other power units may be used as well, e.g. power units that require a connection to the grid.

Thus, the neural (brain) stimulation device may be configured:
- To stimulate the brain, preferably a human brain, electrically and/or transcranially,
- To be mounted on a head of the user for brain stimulation, and/or
- To be operated to stimulate the brain preferably via varying, especially alternating, current stimulation, e.g. tACS or tRNS.

Without to be bound by theory, a frequency or range of frequencies of the alternating current used to stimulate the brain may be adjusted or may be adjustable to a frequency which is e.g. characteristic for the brain rhythm that occurs while performing speech and/or language processing in an area of the brain which is to be stimulated via the varying current. Thus, e.g. tACS in the range of 70 Hz to 80 Hz may be used, e.g. 75 Hz, or within the ranges specified above.

Empirical proof for these frequency ranges was made by performing a corresponding pilot study by the applicant of this patent application, i.e. by Neuro Device Group S.A.

A headband may be comprised in the neural stimulation device. The headband may be elastically or non-elastically. Alternatively, other technical means may be used in order to provide a device that is wearable on the head of the user or patient.

According to an aspect, a neural (brain) stimulation arrangement is provided, comprising:
- The neural stimulation system according to any one of the embodiments mentioned above, and
- The neural stimulation device mentioned above, and
- Preferably or optionally, a user condition monitoring device which is configured to monitor one or more physiological parameters of the user, preferably physiological parameters indicative for the cognitive workload of the user.

Thus, the same technical effects and/or medical effect that apply to the parts of the arrangement may apply to the arrangement as well. The whole arrangement may be wearable by the user/patient. User comfort may be increase by low weight and by using wireless data communication between the parts or components of the arrangement.

Further aspects and embodiments of the disclosure may relate to further functionalities, e.g. to:
- A) A stimulation functionality (see section A below for more details),
- B) A user condition monitoring functionality (see section B below for more details),
- C) An optional session operation protocol Interface (IF) functionality (see section J below for more details),
- D) control unit (CU) (see section D below for more details),
- E) A stimulation planning functionality (see section E below for more details),
- F) A stimulation dose control function 244 (see section F below for more details), and/or
- G) An optional session number tracking functionality (see section G below for more details).

The making and using of the presently preferred embodiments are discussed in detail below. It should be appreciated, however, that the present disclosure provides many applicable concepts that can be embodied in a wide variety of specific contexts. The specific embodiments discussed are merely illustrative of specific ways to make and use the disclosed concepts, and do not limit the scope of the claims.

Moreover, same reference numerals refer to same technical features if not stated otherwise. As far as "may" is used in this application it means the possibility of doing so as well as the actual technical implementation. The present concepts of the present disclosure will be described with respect to preferred embodiments below in a more specific context namely aphasia. The disclosed concepts may also be applied, however, to other situations and/or arrangements as well and/or for other diseases or other speech and/or language improvement situations.

The foregoing has outlined rather broadly the features and technical advantages of embodiments of the present disclosure. Additional features and advantages of embodiments of the present disclosure will be described hereinafter, e.g. of the subject-matter of dependent claims. It should be appreciated by those skilled in the art that the conception and specific embodiments disclosed may be readily utilized as a basis for modifying or designing other structures or processes for realizing concepts which have the same or similar purposes as the concepts specifically discussed herein. It should also be recognized by those skilled in the art that equivalent constructions do not depart from the spirit and scope of the disclosure, such as defined in the appended claims. Although the embodiments are directed to aphasia, other diseases may be treated as well and/or the patient suffering from such a disease may be supported, e.g. Alzheimer disease.

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

The term "disease", as used herein, may refer to an abnormal condition that affects the body of an individual. A disease is often understood as a medical condition associated with specific symptoms and signs. A disease may be caused by factors originally from an external source, such as infectious disease, or it may be caused by internal dysfunctions, such as autoimmune disease. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infectious, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality. In the context of the present invention, the disease may be selected from the group consisting of or comprising aphasia and Alzheimer.

A "stroke" is a medical condition in which poor blood flow to the brain causes cell death.

"Aphasia" is an inability to comprehend and/or formulate and/or produce language because of damage to specific brain regions. The major causes may be stroke and head trauma. Aphasia can also be the result of brain tumors, brain infections, or neurodegenerative diseases, e.g. dementia.

The term "patient", as used herein, may refer to any subject suffering from a disease, in particular suffering from aphasia. The patient may be treated and/or the response to said treatment may be evaluated. The patient may be any mammal, including both a human and another mammal, e.g. an animal. Human subjects as patients are particularly preferred.

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease. "Therapy" may be defined as the attempted remediation of a health problem, usually following a medical diagnosis.

The proposed method and its embodiments may not be used for treatment of the human or animal body by surgery or therapy and may not be a diagnostic method practiced on the human or animal body. Alternatively, the proposed method and its embodiments may be used for treatment of the human or animal body by surgery or therapy and may be a diagnostic method practiced on the human or animal body.

A "medicament/drug" is a drug used to diagnose, cure, treat, or prevent disease. The methods described in this document may be combined with pharmacological intervention, especially for treating aphasia, and/or with motor cortex treatment using electrical stimulation and/or pharmacological intervention. The motor cortex is related to speech because of extensive usage of movable parts of the body, e.g. lips, tongue, vocal cords, respiration, etc. during speaking.

### Brief description of the drawings

For a more complete understanding of the presently disclosed concepts and the advantages thereof, reference is now made to the following description in conjunction with the accompanying drawings. The drawings are not drawn to scale. In the drawings the following is shown in:
- Figure 1: a neural (brain) stimulation arrangement,
- Figure 2: an operation control system (OCS) of the neural (brain) stimulation arrangement,
- Figure 3: a method which is performed by the operation control system,
- Figure 4: parts of a brain stimulation device, and
- Figure 5: results of a pilot study.

### Description of exemplary embodiments

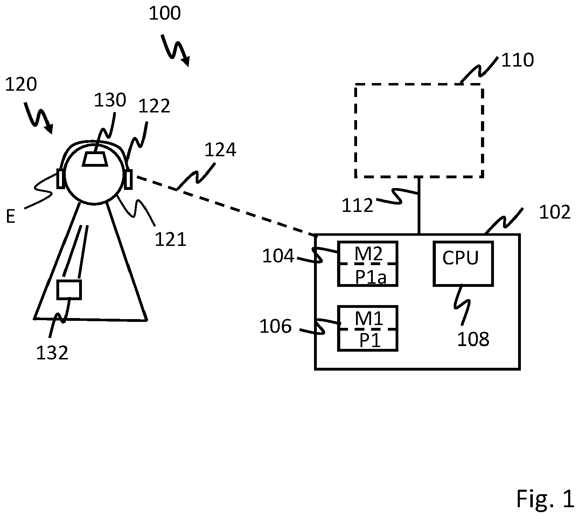

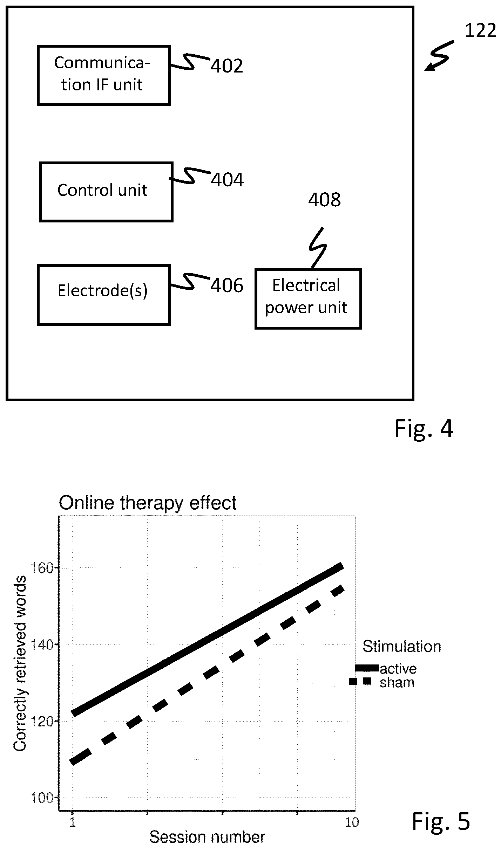

Figure 1 illustrates a brain stimulation arrangement 100 which comprises:
- A brain stimulation system 102, for instance a tablet, a mobile phone, a smartphone or some other kind of computing system, preferably of a portable computing system, performing a control over brain stimulation device 122,
- An optional user interface 110, for instance a touch screen, at least one LED (light emitting diode, at least one button, a loud speaker, etc., e.g. for generating a visible and/or audible output and/or for enabling inputs of a user 120 or patient, and
- A brain (neural) stimulation device 122, e.g. worn on the head 121 of the user 120 and comprising at least one electrode E, 406, see description of figure 4 below,
- An optional user condition monitor device 132.

Brain stimulation system 102 may comprise:
- A volatile memory 104, for instance a RAM (Random Access Memory),
- A non-volatile memory 106, e.g. an EEPROM (Electrically Erasable Programmable Read Only Memory),
- An electronic control unit 108, for instance CPU (Central Processing Unit) or MCU (Microcontroller Unit), and
- Further parts that are not shown in detail, e.g. power source.

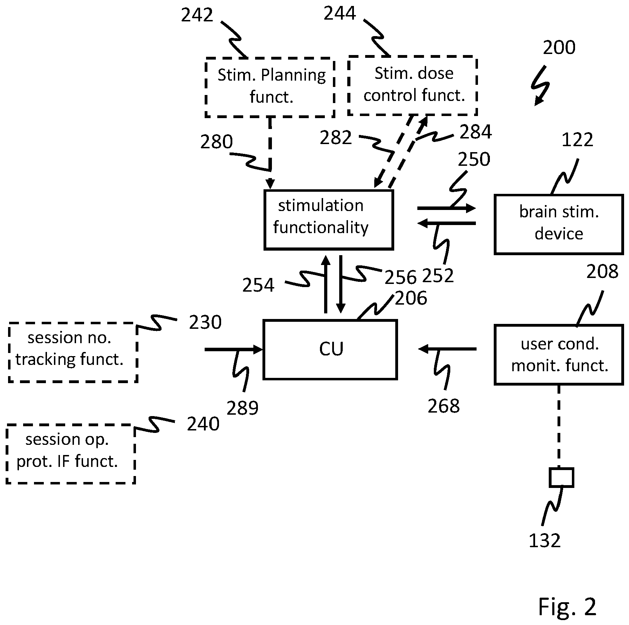

Volatile memory 104 (M2) may store data volatile, i.e. stored data is lost after switching off the brain stimulation system 102. Non-volatile memory 106 may store a program code P1 permanently, e.g. even after switching off the brain stimulation system 102. Program code P1 may comprise a program that realizes functionalities of the brain stimulation system 102. These functionalities are described in more detail below with regard to Figure 2. Non-volatile memory 106 may be an SSD (Solid State Disc) memory, a magnetically storing hard disk or some other kind of memory.

If the brain stimulation system 102 is switched on, program code P1 may be read or copied from non-volatile memory 106 (Ml) as program code P1a and then loaded into volatile memory 104. Volatile memory 104 may allow much faster reading and writing of data than non-volatile memory 106.

Electronic control unit 108 may perform program code P1a, e.g. execute instructions of the program code P1a, and may realize the functions and functionalities of brain stimulation system 102 thereby. Alternatively to a non-volatile memory 106, the program code P1 may be supplied to the volatile memory 104 via a data stream.

Optional user interface 110 may be an integral part of the brain stimulation system 102, which may be a smartphone, a mobile phone a tablet, or it may be a separate device. If user interface 110 is a separate device, there may be a wire connection 112 or a wireless connection between brain stimulation system 102 and user interface 110. Furthermore, a keypad, a pilot remote control or other kinds of input devices may be part of user interface 110, for instance a microphone. User interface 110 may comprise speakers as well. Alternatively or additionally, earphones and/or a microphone may be comprised by or operatively connectable to the brain stimulation device 122 or the user interface 110 for speech input and/or output or for other input, e.g. commands.

User interface 110 may not be necessary or may have a very rudimentary extent, e.g. when most of the functions are realized automatically and/or most of the parameters are set using other possibilities, e.g. preprogrammed by a therapist, medical doctor etc. Alternatively, user interface 110 may be necessary, e.g. when stimulation is started based on demand of user 120 and/or when user 120 has the possibility to set several parameters relevant for stimulation, e.g. length of pauses or extensive planning functionalities as mentioned below.

A user 120 or more specifically a patient, e.g. suffering from aphasia, or another person may wear the brain stimulation device 122 on his or her head 121 to enable electrical stimulation of their brain 130. Relevant for the treatment of aphasia may be especially areas of the brain that may play a role for speech and/or language processing, e.g. language comprehension and/or production, for instance the Wernicke area and/or the Broca area. In context of post-stroke patients, the eloquent areas related to language comprehension and/or production may be naturally shifted to other anatomical areas of the brain due to stroke lesion. Similar phenomena may occur, e.g. in users 120/patients with traumatic brain injury. Brain stimulation device 102 may be configurable in order to stimulate brain areas effectively taking part in language comprehension and/or production and/or speech. Brain stimulation device 122 may be operatively connectable or connected to brain stimulation system 102, e.g. by a wired or by wireless connection 124. Details of brain stimulation device 122 are shown in Figure 4 and are described below.

Optional user condition monitor device 132 may be a device that may have the form of bracelet or a watch and/or that may be carried around the wrist or around another part of the body of user 120. User condition monitor device 132 may comprise a device that measures EDA, i.e. Electro Dermal Activity, of the skin of user 132. Alternatively or additionally, user condition monitor device 132 may include a device for measuring the pulse of user 120 that corresponds to the heart beat of the heart of user 120. Alternatively or additionally, the heart rate variability (HRV) of the heart of user 120 may also be detected or measured. Functionalities of brain stimulation system 102 may use measurement data of user condition monitor device 132, in particular related to the physiological parameters described above, which may give insight into the current arousal/engagement level or cognitive load experienced by the user. In mobile, uncontrolled conditions and without e.g. eye tracking it may be easier to determine the arousal/engagement level compared to e.g. cognitive load or cognitive workload which may be more difficult to be assessed. This is explained in more detail below, see user condition monitoring functionality 208 shown in Figure 2. There may be a wire connection or a wireless connection between user condition monitor device 132 and brain (neural) stimulation device 122 and/or brain stimulation system 102.

User condition monitor device 132 may be optionally used, e.g. when stimulation of the brain 130 of user 120 is started automatically on "implicit" demand of the user, e.g. when user enables the device and the arousal/engagement level of the user 120 rises and when this is detected. Stimulation may also be started automatically, e.g. after the pause, when the arousal and/or engagement level of the user 120 rises and when this is detected. Alternatively, user condition monitor device 132 may be used to stop the ongoing stimulation when the arousal/ engagement level of the user is low for a longer period of time, e.g. 2 mins (minutes), which may be indicative of low demand of speech and language performance support in the current activity, e.g. real-life activity.

Figure 2 illustrates an operation control system 200, OCS of brain (neural) stimulation arrangement 100 of Figure 1. Operation control system 200 may comprise for instance:
- A) A stimulation functionality 202, see section A below,
- B) A user condition monitoring functionality 208, see section B below,
- C) An optional session operation protocol interface functionality 240, see section C below,
- D) A control unit CU, see section D below,
- E) A stimulation planning functionality 242, see section E below,
- F) A stimulation dose control function 244, see section F below, and
- G) An optional session number tracking functionality (see section G below for more details).

There may be more than these functionalities 202 to 244. Alternatively, only some of the functionalities 202 to 244 may be used

### A) Stimulation functionality 202

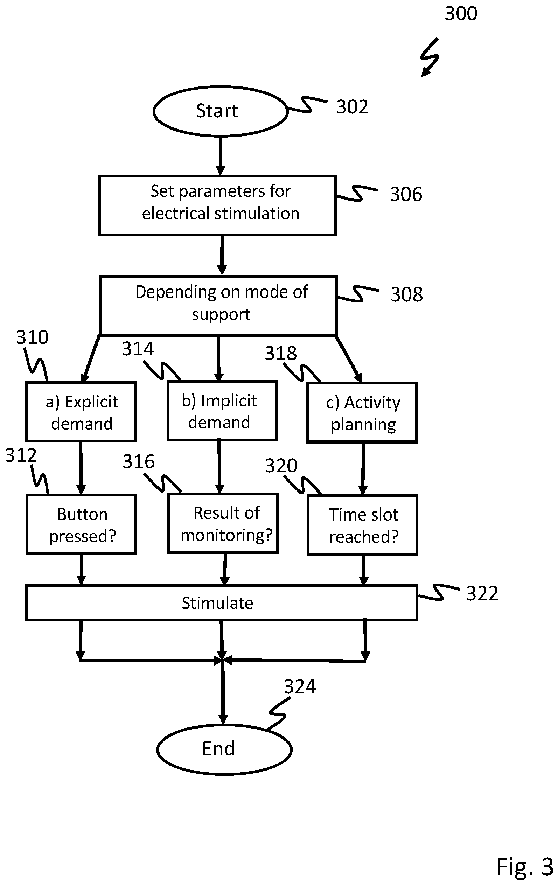

Stimulation functionality 202 may be configured to cause electronic control unit 108 to issue a stimulation command to electrical brain (neural) stimulation device 122 to cause electrical brain stimulation device 122 to perform an electrical brain stimulation procedure. The electrical brain stimulation procedure is described in more detail below with reference to figure 3.

Stimulation functionality 202 may comprise a support stimulation functionality, e.g. for generating tACS signals with a constant frequency. The constant frequency may be a frequency in the range of 60 Hz to 80 Hz or in the range of 70 Hz to 80 Hz, e.g. 75 Hz. A frequency in a much broader range, e.g. 60 Hz to 250 Hz or 70 Hz to 250 Hz may also be used.

The type of stimulation may be contained in the stimulation command to be transcranial electrical stimulation signal in varying current mode, wherein the signal spectrum may consist of only one single frequency from the range mentioned above (i.e. transcranial alternating current stimulation (tACS)). Alternatively, pulses of current of the given frequency may be used. Alternatively, a transcranial random noise stimulation (tRNS) with the frequency's spectrum adjusted to abovementioned ranges may be used.

The current in alternating current mode may have the same or a similar frequency that is also present in the natural oscillation of neurons in the relevant brain areas, e.g. brain areas that are relevant for language processing and/or production.

The stimulation command may include stimulation data. Alternatively or additionally, the stimulation command may refer to stimulation data that is stored in memory, for instance in memory 104 or 106. Reference is made to the introductory part of this description for the details of the stimulation data and the stimulation parameters that may be comprised in stimulation data, for instance amplitude and frequency of AC (alternating current) current, start and duration of electrical brain stimulation, etc.

There may be device related stimulation commands that may be specific for brain stimulation device 122. Alternatively or additionally, stimulation commands on a higher level may be used between the functionalities of operation control system 200.

### B) User condition monitoring functionality 208

Operation control system 200 may comprise the user condition monitoring functionality 208 which may be configured to determine, based on user condition data that is indicative for the current condition of user 120 whether the support is required and/or whether the support should be continued.

The user condition data may be data that is indicative for the current arousal and/or engagement level or cognitive load (cognitive workload) experienced by the user 120 and/or it may comprise data on one or more physiological parameters of user 120 which may be monitored by the neural (brain) stimulation system 102 before starting and/or during a support stimulation phase.

If the support stimulation phase should be started, operation control system 200 may be configured to cause electronic control unit 108 to issue a start simulation command or another appropriate command in order to start, the brain support stimulation phase.

If the support stimulation phase should be stopped or interrupted, operation control system 200 may be configured to cause electronic control unit 108 to issue a stop stimulation phase command or an interrupt stimulation phase command to stop, preferably end, or to interrupt, preferably only temporarily, the support stimulation phase.

There may be the following possibilities to determine the end of the support stimulation phase:
- A duration of the stimulation may be used that has always the same length, for instance a duration of less or equal to 15 minutes, and/or
- The physiological parameters are indicative of low arousal and/or engagement level, i.e. they are below thresholds that are set for user 120 or that are user independent, and/or
- The user 120 signals that he or she does not need the support stimulation anymore, e.g. by pressing a button and/or in another appropriate way.

The brain stimulation phase may be stopped if a phase duration limit or a session duration limit is reached. The phase duration limit or the session duration limit may depend on user 120 or may be user independent. Furthermore, termination of brain stimulation phase may be possible if user condition is too bad and/or if a number of interruptions is higher than a threshold set for user 120 or independent of user 120.

### C) Session operation protocol interface functionality 240 (optional)

The session operation protocol interface functionality 240 of brain stimulation system 102 may be used to receive, preferably user-specific, stimulation phase operation data that may be used for support stimulation, e.g. the following data:
- One or more values of stimulation parameters for the stimulation phase which is to be performed or for the stimulation phases which are to be performed. Examples for parameters are stimulation frequency, amplitude of current, overall length of stimulation per day or other reference frame, length of pauses, maximum length of one stimulation phase, etc.

An authentication procedure may have to be successfully completed by a practitioner or other person who is different from user 120 who is made or will be made subject to the brain stimulation session in order to get access to the session operation protocol interface functionality 240. The authentication procedure may prevent misuse of the neural (brain) stimulation device 102 and/or may guarantee that safety and/or health of the user 120/patient is not jeopardized by wrong parameters.

### D) Control unit CU

The control unit CU may be configured to control the electrical stimulation unit 202 depending on the stimulation parameters, e.g. length of a pause or of several pauses having constant length, activation planning, etc., see e.g. functions of control unit 108, CU mentioned in the introductory part of this document and/or in the appending claims.

Control unit CU may coordinate and/or synchronize stimulation functionality 202 and stimulation planning functionality 242 and/or stimulation dose control function 244.

Control unit CU may operate dependent on inputs delivered by user condition monitoring functionality 208, e.g. based on inputs that are generated based on outputs of user condition monitoring device 132 or that are generated in another appropriate way in order to assess e.g. whether the user 120 needs support stimulation in order to enhance their speech and/or language capabilities in real time, i.e. without significant delay between electrical (external) brain stimulation and improvement of the language processing skills.

Control unit CU may collect all relevant data that is generated during the operation of brain stimulation system 102. This data may be used for later evaluation or for other purposes.

### E) Stimulation planning functionality 242 (optional)

The stimulation planning functionality 242 may be used if the user 120 intends to perform activities that last longer than a maximum allowed stimulation dose (duration) or stimulation budget. To facilitate such activities, the neural (brain) stimulation system 102 may be equipped with a stimulation planning functionality 242, wherein the user 120 may input information on anticipated start time and duration of activities during e.g. the next 24 hours or other time interval and the neural (brain) stimulation system 102 may automatically distribute the stimulation dose or the stimulation budget over a predefined period (reference time interval or reference time frame) in order to maximize stimulation outcomes and not to exceed the total maximum stimulation time (dose, budget). This may be achieved by adjusting length of stimulation phases and/or length of pauses between stimulation phases.

Stimulation planning functionality 242 may be similar to MS (Microsoft, may be a trademark) Outlook calendar or similar to another personal organizer program or scheduler. Alternatively, stimulation planning functionality 242 may be coupled to MS (Microsoft, may be a trademark) Outlook calendar or to another scheduler program.

The stimulation planning functionality 242 may be used in combination with manual or explicit demand for electrical brain stimulation and/or with automatically implicit activation on demand depending on the state of the user. Thus, manual or automatically activation may be enabled only within the planned timeslots. Alternatively, manual or automatically activation may be enabled within the planned timeslots and also without the planned timeslots, e.g. thereby reducing stimulation already planned budged or using an additional budget.

Alternatively, stimulation on demand (explicit or implicit) may be disabled if the stimulation planning functionality is used or enabled.

### F) A stimulation dose control function 244 (optional).

The stimulation dose control function 244 may measure or may detect the time at which support stimulation is performed on the brain 130 of user 120, e.g. within a reference time frame. The reference time frame may be on day, i.e. 24 hours, or another appropriate time interval.

The stimulation dose control function 244 may compare the measured or detected time value with a reference value that corresponds to the maximal dose (budget). If the accumulated measured or detected value is lower than the value of the maximal dose, further support stimulation may be enabled. If the accumulated measured or detected value is equal to and/or higher than the value of the maximal dose, further support stimulation may be disabled. Thus, health of the user 120 may be preserved by not using too long overall stimulation time which could also have detrimental effects for enhancing speech and/or language processing capabilities of user 120.

The electrical signal used for stimulation may have a constant amplitude value, e.g. a value that may be established either by an amplitude tested in clinical trial or assumed based on the required electric field. Thus, effective stimulation may be performed. A valid criterion for selecting the amplitude value may be to find and/or to use the lowest current value that is effective to improve speech and/or language processing skills.

### G) Session number tracking functionality 230 (optional)

The session number tracking functionality 230 of brain stimulation system 102 may be configured to track or count the number of stimulation phases and/or days on which stimulation phases are used by user 120. When the number of sessions and/or stimulation phases has reached a predetermined maximum, the operation control system 200 may be configured to prevent initiation of a subsequent support stimulation phases/sessions. This function may also play a key role for billing and/or accounting the service that is supplied by brain stimulation system 100. A session may comprise all the simulation phases during one reference time frame, e.g. 24 hours.

The brain stimulation system 102 and/or the software that is comprised in brain stimulation system 102 may be distributed as a service. Prepaid service for a fixed number of stimulation phases may be used. Session number tracking functionality 230 or another dedicated functionality may count up the number of stimulation phases and/or of sessions and may block the further use of the brain stimulation system 102 if the number exceeds the number of session/phases that have already be paid for. Other payment systems and/or methods are possible as well, for instance pay by credit card, pay by session, pay by stimulation phase, etc.

If the further use of brain stimulation system 102 is blocked, it may be necessary to involve a practitioner or physician for adjusting or confirming the parameters that are used for support stimulation. After the involvement of the practitioner or physician it may be possible to unlock brain stimulation system 102 again in order to perform further stimulation phases, for instance by prepaying and or by using other methods of payment.

There may be the following relations or connections between functionalities 202 to 244:
- A relation 250 between stimulation functionality 202 and brain stimulation device 122 that may be used for instance for transmission of first or lower level stimulation commands and/or stimulation data,
- A relation 252 between brain stimulation device 122 and stimulation functionality 202 that may be used for instance for transmission of confirmation messages which indicate that lower level stimulation commands have been received and/or are being or have been executed properly,
- A relation 254 between control unit CU and stimulation functionality 202 that may be used for instance for transmission of first level stimulation commands or higher level stimulation commands and/or stimulation data which may be on a higher protocol level than commands that are transmitted via relation 250, for instance a stop session command, an interrupt session command, etc.,
- A relation 256 between stimulation functionality 202 and control unit CU that may be used for instance for transmission of confirmation messages which indicate that higher level stimulation commands have been received and/or are being or have been executed properly,
- A relation 268 between user condition monitoring functionality 208 and control unit CU that may be used for instance for transmission of measured values of physiological parameters of user 120, for instance EDA and/ or pulse frequency, blood pressure etc. The values of physiological parameters may be part of user condition-specific-data. User condition monitor device 132 may deliver these data.
- a relation 280 that may be used to transfer planning data to the stimulation functionality/ unit 202,
- A relation 282 that may be used to disable stimulation functionality/ unit 202 if the maximal stimulation dose has been reached or that may be used to transfer a stimulation stop command if the maximal stimulation dose has been reached, and
- A relation 284 that may be used to confirm that a stimulation stop command has been received or for other purposes, e.g. transmitting overall support stimulation time value or transmitting data indicating when a support stimulation phase starts and/or data indicating that support stimulation is active (e.g. send in periodic time intervals) and/or data indicating that a support stimulation phase is ended.

A local input interface 289 may be used by a practitioner or other person in order to input relevant data.

The relations 250 to 284 may be realized as connections or other means for data transmission between software modules, e.g. transfer buffers, etc.

Figure 3 illustrates a method 300 which may be performed by the operation control system (OCS) 200 that is shown in Figure 2 or by any other appropriate system. All or only some of the functionalities 202 to 244 may be used to perform the steps of method 300. The method may start in step 302 for instance by pressing a button, e.g. a switch-on, switch-off button.

In step 306, parameters for the electrical support stimulation of the brain of user 120 may be set, e.g. during manufacturing of brain stimulation system 102 and or thereafter. Thus, the parameters for the electrical support stimulation of the brain of user 120 may be set by a practitioner or by a physician based on a diagnosis made for a disease that user 120 may have, e.g. also depending on the degree of the disease, e.g. of aphasia. Alternatively, user 120 may not have a disease but may want to improve its speech and/or language skills. Also for this use case, a skilled person or user 120 may be allowed to set all or some of the parameters.

Up to a limited extend, it may also be possible that user 120 sets parameters, e.g. parameters specifying the length of pauses and of stimulation phases, or the overall stimulation time within a reference time frame, e.g. within upper limits that are determined as safe and/or lower limits that are known to provide impact on improvement of speech and/or language skills.

A step 308 may follow after step 306. Step 308 may enable to select manually or automatically at least one of the following support modes a), b) and c).

Alternatively or additionally, other support modes may be selected in step 308. Further, alternatively only one support mode may be possible, i.e. selection of one of several modes is not possible.

### a) Deliver support stimulation on explicit demand of user 120

Optional step 310 may be performed if explicit support mode a) is activated. Variables may be set and/or buttons or switches may be monitored or activated that may be used to signal that support stimulation is needed by user 120.

A step 312 may follow after step 310. In step 312 it is checked whether the user 120 demands support stimulation or corresponding user input of user 120 is received. After detecting explicit user input, the left stimulation budget may be checked, e.g. using stimulation dose control functionality 244. If there is enough budget or if budget has not to be considered, support stimulation may be started, see step 322.

According to another embodiment, user 120 may alternatively and/or additionally also decide in support mode a) when he does not need support stimulation anymore, e.g. by providing corresponding user input via user interface 110 or via another (simpler) user interface.

### b) Deliver support stimulation depending on monitored state of user 120 (implicit demand)

Optional step 314 may be performed if implicit support mode b) is activated. Variables may be set and/or user condition monitoring functionality/unit 208 may be activated in order to monitor the condition or state of user 120.

A step 316 may follow after step 314. In step 316 condition or state of user 120 is monitored. After detecting that user 120 needs support stimulation, the left stimulation budget may be checked, e.g. using stimulation dose control functionality 244. If there is enough budget or if budget has not to be considered, support stimulation may be started, see step 322.

### c) Using activity planning to deliver support stimulation

Optional step 318 may be performed if planning support mode c) has been selected in step 308. Control unit CU and/or stimulation planning functionality 242 may be activated to consider planned time slots before starting and/or enabling support stimulation. Planning of the time slots may took place before starting method 300 or may be part of step 302, e.g. start of method 300. As mentioned already above, stimulation planning functionality 242 may be used to plan support stimulation or to enable support stimulation within time slots that may be selected e.g. by user 120.

A step 320 may follow after step 318. In step 320 it may be checked, whether a planned time slot has been reached, e.g. by control unit CU and/or by stimulation planning functionality 242. If a planned timeslot has been reached, stimulation may be started, see step 322. Alternatively, condition monitoring of user 120 may be started in order to deliver support stimulation on implicit demand. Alternatively or additionally, manual (explicit) demand by user 120 may be activated in order to start stimulation on explicit demand of user 120 only during the planned time slots.

In step 322, the support stimulation is performed. In support mode a) but optionally also in support mode b) and c) the user 120 may decide when they do not need support stimulation anymore, e.g. by providing corresponding user input via user interface 110 or via another (simpler) user interface. If user 120 signals that they do not need stimulation anymore, stimulation made in step 322 may be stopped.

Especially, in support mode b) but optionally also in support mode a) or c) user condition monitoring data may be checked in step 322 to assess whether user 120 still needs support stimulation. If not, stimulation in step 322 may be ended.

In support mode c), the stimulation of step 322 may also be ended if the end of a planned time slot is reached.

The end of a support stimulation phase may be followed by a pause and thereafter stimulation may be possible again, e.g. according to the activated support mode.

The end of method 300 may be reached in step 324, e.g. by switching off neural (brain) stimulation system 102. A further reason to end the method 300 may be that the overall stimulation budget for the reference time frame has been reached. In this case further support stimulation may not be possible until the start of the next reference time frame.

Optionally, method 300 may comprise steps that use other functions (auxiliary functions) mentioned above, e.g. tracking stimulation phase number, accounting steps, e.g. checking whether payment for further stimulation is made in prepaid mode, etc.

Figure 3 illustrates a flowchart of a computer program product, e.g. software, according to the present disclosure. It will be understood that each block or step of the flowchart and combinations of blocks in the flowchart can be implemented by computer program instructions. These computer program instructions may be loaded onto a data processing unit or another programmable apparatus to form a machine such that the instructions which are executed on the data processing unit or other programmable apparatus create means for implementing the function specified, especially when executed on the data processing unit or other programmable apparatus, in the blocks or steps of the flowchart. These computer program instructions may also be stored in a computer-readable memory that can direct a data processing unit or other programmable apparatus to function in a particular manner. Moreover, these computer program instructions may be downloaded in e.g. a telecommunications network to cause operational steps to be performed on the data processing unit or other programmable apparatus to produce a computer implemented process such that the instructions which execute on the data processing unit or other programmable apparatus provide steps for implementing the functions specified in the blocks or steps of the flowchart. Accordingly, blocks or steps of the flowchart support combinations of means for performing the specified functions, combinations of steps for performing the specified functions and program instruction means for performing the specified functions.

It will also be understood that each block or step of the flowchart and combinations of blocks or steps in the flowchart can be implemented by special purpose hardware-based computer systems which perform the specified function or steps or combinations of the special purpose hardware and computer instructions.

Moreover, dedicated circuitry without using a processor may be used to perform method steps 302 to 324 of method 300, e.g. a finite state machine.

There may be less method steps than shown within figure 3. Alternatively there may be more method steps to realize all functions of brain stimulation system 102. Furthermore, the sequence of some of the steps may be changed.

Figure 4 illustrates units of the brain stimulation device 122. The brain stimulation device 122 may comprise:
- A communication interface 402,
- A control unit 404, for instance an MCU (Microcontroller Unit) or a CPU (Control Processing Unit),
- At least one electrode E, 406 or at least two electrodes 406, or three or more electrodes, where, preferably, at least one of the electrodes 406 is configured to be arranged on the head of the user 120 and the other electrode E, 406 may be placed for instance on an appropriate location of the body of user 120, for instance on his or her contralateral side of the head, or on a shoulder or on an arm, and
- An electrical power supply unit 408, for instance a battery or an accumulator or other appropriate power supply, e.g. connected to the power grid.

Communication interface 402 may be a wireless interface, for instance a Bluetooth interface. Stimulation commands may be received by brain stimulation device 122 from brain stimulation system 102 via communication interface 402, especially via a receiving unit of the interface (not illustrated). The communication interface 402 may comprise a transmitting unit. Confirmation messages may be sent from communication interface 402 to brain stimulation system 102 using the transmitting unit.

The control unit 404 may control and coordinate the processing of stimulation commands within brain stimulation device 122. The at least one electrode E, 406 or the at least two electrodes 406 may be connected to appropriate output circuitry. The control unit 404 may send signals or data to this output circuitry according to the stimulation commands. Thus, it may possible to generate constant current output or varying current output at electrode(s) E, 406 as desired. A gel or saline solution may be used to improve current transmission between the electrodes 406 and the cranium and/or brain 130 of user 120. Alternatively, dry electrodes may be used. Electrical power supply unit 408 may supply electrical energy to other units of brain stimulation device 122.

Various electrode montages/arrangements may be used. In general, at least one electrode (stimulating electrode) may be located close to areas of the brain 130 responsible for speech and language functions. In most users 120/patients these areas may be Wernicke and/or Broca areas. However, in some post-stroke patients or patients with severe traumatic brain injury, the anatomical Broca and/or Wernicke areas may be damaged and new areas of the brain may take over speech and language processing. The stimulation target may be selected individually for patients, e.g. using fMRI (functional Magnetic Resonance Imaging) mapping. Most common (but not all possible) positions of stimulating electrode(s) following the 10-20 system (commonly used for EEG and brain stimulation and invented by Herbert Jasper) may be: C3, C5, FC3, FC5, CP3, CP5, P5, PO7.

There may be at least the following options for positioning of counter electrode:
a) Contralaterally on head - i.e. on the right side of the head in case of stimulation electrode positioned on the left side of the head (over Wernicke/Broca areas),
b) Contralaterally and extracephalically - e.g. on the right arm or shoulder, and/or
c) HD(high density)-tACS montage or arrangement, wherein at least one stimulating electrode may be located over the stimulation target and wherein there may be multiple, e.g. 3, 4 or more, counter electrodes located around the at least one stimulating electrode, in distance larger than e.g. 2 cm.

The brain stimulation device may provide output current sufficient to induce electrical field in the target area of the brain higher than 0.08 V/m, preferably at least 0.2 V/m, but lower than e.g. 0.3 V/m. The peak-to-peak amplitude of the output current may be preferably 2 mA (+/- 1 mA) or within a range of 1 mA to 4 mA (+/- 0.5 mA to +/-2 mA).

The brain stimulation device may provide compliance voltage sufficient to generate above mentioned current amplitude considering impedances of electrodes used. E.g. gel or saline soaked electrodes may have impedance of 3 kOhm (kilo Ohm) to 10 kOhm. Alternatively, dry electrodes may be used, operating with impedance of 15 kOhm to 20 kOhm. Hence, the brain stimulation device may provide compliance voltage of at least 20 V, but e.g. not higher than 48 V.

According to an embodiment, the neural (brain) stimulation system 100 may comprise the user condition monitoring unit 208 configured to determine a state of the user 120. The neural stimulation system 100 may be configured to stop stimulation, e.g. without knowledge of the user 120, or to generate a request to the user 120 in order to stop stimulation if a decreased arousal and/or engagement or an decreased cognitive load is detected based on the determined state of the user 120. Preferably, the neural stimulation system 100 may be configured to generate a request to the user 120 whether they want to stop the stimulation. Preferably the stimulation may be stopped if the user 120 decides to stop the stimulation in answer to the request.

Alternatively or additionally the neural stimulation system 100 may be configured to stop stimulation if a condition or state of the user 120 is indicative of a pathological state of the user 120.

According to a more detailed embodiment, the neural (brain) stimulation system 102 may comprise:
- The user condition monitoring unit 208 for detecting a state of the user 120, e.g. mental, physical, etc., and
- The control unit CU that may be configured to stop the stimulation automatically depending on the detected state, e.g. without knowledge of the user 120, or to generate automatically a request to the user 120 in order to stop the stimulation depending on the detected state, preferably if a decreased arousal and/or engagement or an decreased cognitive load is detected based on the determined state of the user 120 or if a condition or state of the user 120 is indicative of a pathological state of the user 120.

The user condition monitoring functionality 208 may be configured to determine user condition data indicative for the current condition of the user 120 experienced by the user 120 and/or one or more physiological parameters of the user 120 which may be monitored by the brain stimulation system 102 during performing the speech and language related activities by the user 120.

At least one of the user condition data and/or the one or more physiological parameters is used or are used to stop the stimulation when the user 120 experiences decreased arousal and/or engagement level or cognitive load in order to stop the support of the user 120 if the user does not need the support stimulation in the performed activity anymore.

Preferably, the user condition data may be data indicative for the current arousal and/or engagement level and/or cognitive load of the user 120, e.g. fatigue and/or mental load.

Preferably, the one or more physiological parameters of the user 120 may be based on electrodermal activity and/or on the pulse frequency and/or heart rate and/or blood pressure of the user 120 and/or on heart rate variability of the heart of the user 120 and/or blood oxygenation level of the blood of user 120.

In other words, a supportive system for aphasic patients is disclosed.

The disclosure relates e.g. to a brain stimulation system 102, especially to a brain stimulation system 102 that may be used to support daily activities of a patient 120 suffering from aphasia.

One object of this application may be to introduce a brain stimulation system 102 being configured to provide supportive and assistive function, allowing the user 120 to improve speech and/or language performance, e.g., the user 120 suffering from aphasia, when the user 120 intends to perform activities requiring challenging and/or demanding (intensive) language performance in their daily life and the brain stimulation system 102 intends to boost this performance.

Exemplary use cases may be the following:
1. The user 120 may plan to attend a meeting, wherein they would like to actively take part. Due to aphasia, the user 120 normally has difficulties with fluent communication, thus limiting their active participation in a conversation. The user 120 may put on the brain stimulation system 120 and may turn it on when the meeting is about to start. The meeting may be scheduled for a specific amount of time, thus the brain (neural) stimulation system 102 may automatically distribute a predefined stimulation dose (budged) through the entire anticipated meeting time. The brain stimulation system 102 may facilitate speech production, thus allowing for participation with increased speech-language performance, what would result in better communication and more satisfying social contact.
2. The user 120 may plan to go shopping and may need to ask on details of products they intend to purchase. Due to aphasia, their capabilities of communication with shop's employees would be limited. The user 120 may turn on the brain stimulation system 102 just before entering the shop. The brain stimulation applied to the brain 130 of the user 120 may facilitate communication. Thus the user 120 may effectively obtain the required information from the shop's employees. The experience of the user 120 in daily activities may be more satisfactory.

The disclosed brain stimulation system 120 and the disclosed method may be based on findings on positive effects of tACS (transcranial alternating current stimulation) or other electrical brain stimulation on speech-language performance during the time of introducing (applying) the stimulation to a person with aphasia, e.g. an online effect, real-time effect.

NDG (Neuro Device Group S. A.) has performed a pilot study with 75 Hz (Hertz) tACS (transcranial alternating current stimulation) paired with performing speech-language tasks by the patient/user 120. While patients/users 120 received active 75 Hz tACS, and the task was to name pictures without any cues, they were able to name correctly more items in a fixed length of the therapy session (total of 40 mins (minutes)) as compared to sham stimulation.

### Description of the pilot study and the results:

The aim of the pilot, randomized, crossover study was the feasibility and efficacy assessment of therapeutic effect of the tACS in a group of post-stroke patients with chronic aphasia.

10 persons with aphasia, who were more than 6 months post stroke completed two phases of experimental therapy. In the experimental phase patients received 10 therapeutic sessions comprising of language training (naming ability) paired with 75Hz tACS. In the control phase, patients received 10 therapeutic sessions comprising of language training (naming ability) paired with sham stimulation. The order of phases was randomized - tACS/sham, or sham/tACS.

In this study participants trained only those words that they were unable to name correctly during baseline measurement. The language training followed the vanishing cues framework rules to ensure best possible improvements and maintain a high level of participants' motivation. Vanishing cues were implemented via 5 presentation levels of tasks. The first level included a picture to be named by a participant with 3 cues: the name of the word, audio recording and video recording with the pronunciation of the word. If answer was correct, the number of cues was reduced on each level. On the last, fifth level patient was asked to name the object on the picture without any additional support, so the task on this level refers to pure naming.

The online therapy effect is defined as number of correct answers on the fifth level. Patient's performance on fifth level of therapy task represents the naming ability, or word retrieval ability in a more general context. Both are crucial for satisfactory use of language in real life situations.

### Online therapy effects

To verify the effect of neuro stimulation during the therapy session the multilevel (mixed effects) modeling has been performed. The model was constructed using the lme4 package in R. Further information on the lme4 package including examples of its usage are known e.g. from Bates et al., "Fitting Linear Mixed-Effects Models Using lme4", Journal of Statistical Software, October 2015, p.1 to p. 48. The lme4 package of "The R project for Statistical Computing" may be found at e.g. https://cran.r-project.org/web/packages/lme4/lme4.pdf on December 22, 2022. Both documents are included by reference in this document for all legal purposes.

Data were modeled by type of intervention (tACS or sham), therapeutic session number and the interaction of these fixed effects. Random effects were estimated for patient and therapeutic session number (with the former being nested within the latter). Dependent variable defined as 'number of correct answers on the last level' will be served as a therapy progress indicator. Intercept for average performance is placed in the middle session (i.e. 5th out of 10 sessions in a phase, either with tACS or sham stimulation).

Data yielded evidence in favor of a main effect of stimulation, which is significant and positive ((beta = 4.44, 95% CI [2.04, 6.84], t(189) = 3.63, p < .001; Std. beta = 0.06, 95% CI [0.03, 0.10]), where beta is an estimate for the fixed effect (contrary to random effect that have also be considered in the model), CI is the confidence interval, t is the value with the degrees of freedom, p is the probability of a null hypothesis (e.g. that there are no differences in the number of correctly retrieved words on the 5th level between 75 Hz tACS and sham (dummy) stimulation conditions) and "Std." refers to the standard deviation.

This effect shows that active stimulation is significantly (p<0.001, meaning that the null hypothesis or no-effect hypothesis can be rejected) more effective than sham stimulation. It means that patients were able to retrieve more words, when they were stimulated with 75Hz tACS, compared to sham (see figure 5).

Figure 5 illustrates the results of the pilot study using a Cartesian coordinate system. The horizontal x-axis relates to the session number in the range of 1 to 10. The vertical y-axis relates to the "correctly retrieved words" within the range of 100 to 170.

The upper line relates to "active" support stimulation. The upper line extends from about 122 correctly retrieved words in session number 1 to about 161 correctly retrieved words in session number 10.

The lower line relates to sham stimulation. The lower line extends from about 109 correctly retrieved words in session number 1 to about 155 correctly retrieved words in session number 10.

Thus, there is a significant difference between "active" and sham stimulation, resulting e.g. into an improvement of about 12 percent in the first session and still in an improvement of about 4 percent in the tenth session.

More precisely, when patients were receiving active stimulation, they were able to name correctly more pictures (presented without any cue).

It's worthy to highlight that patients trained only those words, which they could not name, or had major difficulties with, during the baseline measurement (measured before first session).

Data also revealed a main effect of the session, which is statistically significant and positive ((beta = 4.67, 95% CI [3.13, 6.22], t(189) = 5.93, p < .001; Std. beta = 0.18, 95% CI [0.12, 0.24]).

The main effect of the session (p<0.001) shows that patients achieve better results along the course of the 2-weeks long therapy. It can be interpreted as a classical training effect - patients got familiarized with the task and learned new words, so the execution was more and more effective.

There was no evidence of an interaction of treatment over time, which might suggest that stimulation effects were rather constant over 2-weeks long therapy.

### Conclusions from the pilot study

The data, as well as subjective opinions of study's participants, suggest that 75 Hz tACS facilitates speech and language performance in real time. Unlike in a scenario with use of the brain stimulation system 102 to improve training outcomes, in this application we disclose the system 102 for online use (e.g. meaning during the stimulation), wherein the user 120 may perform daily activities instead of performing specific training tasks. The brain stimulation system 102 may provide supportive and assistive function at the moment when the electrical neural (brain) stimulation is delivered, e.g. synchronously with the stimulation. A generalization of the result may be justified up to a certain extend due to basic knowledge of neuroscience. Thus, similar results may be expected and/or achieved with other frequencies than 75 Hz, especially with "nearby" frequencies, e.g. within a range of minus 20 percent to plus 20 percent of 75 Hz, within a range of minus 10 percent to plus 10 percent of 75 Hz, within a range of minus 5 percent to plus 5 percent of 75 Hz, and or within the same brain wave type, e.g. gamma waves. Low gamma wave band may be defined in the range of 30 Hz to 60 Hz or 30 Hz to 70 Hz and high gamma range may be defined in the range of 61 Hz to 260 Hz or 71 Hz to 250 Hz depending on the definition value of the border between low gamma range and high gamma range of brain waves.

### Further issues

In an embodiment, the neural (brain) stimulation system 102 may be configured to deliver non-invasive electrical brain stimulation to speech and language related brain areas at the moment when the user intends to increase his/her speech-language performance. In essence, it is up to the user 120 to decide when the stimulation shall be used.

The brain stimulation, especially the brain stimulation system 102, may use 75 Hz tACS which may be the same frequency that was used in study run by Neuro Device Group S.A. (NDG). However, knowledge of brain functioning may suggest that any stimulation frequencies falling into the range of high-gamma, e.g., a one frequency (single frequency value) selected from the range of 70Hz to 250Hz, may have similar effects, as high-gamma band is characteristic for speech production (Gaona et al., "Nonuniform High-Gamm (60-500 Hz) Power Changes Dissociate Cognitive Task and Anatomy in Human Cortex", The Journal of Neuroscience, February 9, 2011). Specifically, a prominent power increase was observed between 60 Hz to 100 Hz in superior temporal gyrus, thus stimulation within this frequency band might tune up the brain of a person with aphasia, what may result in more effective speech production.

Due to safety concerns tACS may not or shall not be delivered for prolonged periods of time. Providing that it is a user 120 who decides when to use the brain stimulation system, the system may be equipped with stimulation dose control functionality limiting the total time of active stimulation within 24 hours, i.e. the function may not allow to exceed a total maximum stimulation time. For example, the total maximum stimulation time may be 60 mins of stimulation in 24 hours or within another reference frame.

The user may intend to perform activities that last longer than the maximum allowed stimulation dose or stimulation budget. To facilitate such activities, the neural (brain) stimulation system 102 may be equipped with a stimulation planning functionality 242, wherein the user 120 may input information on anticipated start time and duration of activities during e.g. the next 24 hours or other time interval and the neural (brain) stimulation system 102 may automatically distribute the stimulation dose or the stimulation budget over predefined period (time interval) in order to maximize stimulation outcomes and not to exceed the total maximum stimulation time (dose, budget).

In an additional embodiment, the neural (brain) stimulation 102 system may comprise a user condition monitoring functionality 208 that may be configured to determine user condition data indicative for arousal and/or engagement level and/or cognitive load of a user. These measures may be obtained based on one or more physiological parameters of the user 120, e.g. electrodermal activity and/or on the pulse frequency and/or heart rate (HR) and/or blood pressure of the user 120 and/or on heart rate variability (HRV).

Combined with a stimulation dose control functionality 244 and/or stimulation planning functionality 242, the user condition monitoring functionality 208 may support optimization of active stimulation periods during performing of activities by the user 120. For example, a low cognitive workload may be indicative of low requirements of the user 120 to support their performance. Thus, the stimulation at the moments with low cognitive workload may not be necessary for accurate performance and the brain stimulation functionality 202 may be inactive.

However, when the neural (brain) stimulation system 102 observes increasing arousal/engagement level or cognitive workload it may be assumed, that the user 120 commenced on performing portions of activities that may be more difficult due to limited speech and language capabilities resulting from aphasia or due to other restrictions of their speech and/or language processing capacity. In such case, the neural (brain) stimulation system 102 may automatically start stimulation to support user's performance. Alternatively, the neural (brain) stimulation system 102 may automatically suggest the start of stimulation to the user 120. The user 120/patient may accept the start in order to start stimulation. If the user 120/patient does not accept, stimulation is not started. For safety reasons or other reasons, e.g. regulatory reasons, the neural (brain) stimulation system 102 may wait for the approval of the user 120/patient before starting the stimulation.

For example, each time after each start of stimulation program by the user 120, the stimulation functionality 202 may be turned on for a specific period of time, e.g. 10 mins. After this initial period the neural (brain) stimulation system 102 may turn the stimulation functionality off and may keep monitoring user condition data. When the user condition data are indicative of increasing cognitive workload, the system may resume (continue) stimulation for a certain time (e.g. another 10 mins) or until user condition data are indicative of decreased or low arousal and/or engagement level or cognitive workload.

Alternatively, when the user condition data are indicative of increasing arousal level and/or engagement level or cognitive workload, the system may suggest to the user to resume (continue) stimulation for a certain time (e.g. another 10 mins) or until user condition data are indicative of decreased or low arousal/ engagement level or cognitive workload.

Therefore, increased arousal/engagement level or cognitive load may be interpreted as an implicit demand of the user 120 for support.

Computerized speech language therapy or language support may be tailored by use of novel technological solutions, especially for detecting the internal state of the user 120/patient:
- Electrodermal activity,
- Heart rate variability,
- Pulse.

A method of how to assess cognitive load from psychophysiological measures is described in the paper by Eija Haapalainen et al., "Psycho-Physiological Measures for Assessing Cognitive Load", Behavioural Brain Research 293 (2015) 2 UbiComp ' 10: Proceedings of the 12th ACM international conference on Ubiquitous computing, September 2010, Pages 301-310. The content and methods described in this paper are incorporated by reference herewith.

In the paper by Henrik Wiberg et al., "Physiological responses related to moderate mental load during car driving in field conditions", Biological Psychology, Volume 108, May 2015, Pages 115-125, multiple indicators, like heart-rate, skin conductance level, breath duration, blink frequency, blink duration, and eye fixation related potential and self-reporting were used to determine the cognitive load. The content and methods described in this paper are incorporated by reference herewith.

In the paper by Charlotte Larmuseau et al., "Combining physiological data and subjective measurements to investigate cognitive load during complex learning", Frontline Learning Research, 7(2), May 2019, Pages 57-74, it was studied how physiological data, namely electrodermal activity (EDA) is related to mental effort. The content and methods described in this paper are incorporated by reference herewith.

According to an embodiment the size of electrode(s) and/or the shape of electrode(s) and/or the arrangement of the electrodes may be as follows:
- E.g. square or rectangular electrodes with skin contact area e.g. in the range of 16 cm² (square centimeters) to 35 cm² may be used,
- E.g. round electrodes with the radius e.g. in the range of 1.5 cm to 2.5 cm (centimeters) may be used,
- E.g. HD-tACS (High Definition tACS) montage may be used comprising multiple e.g. round electrodes of e.g. radius around 0.5 cm. In this type of montage there may be one stimulating electrode located over the stimulated brain area and multiple (e.g. 4 or in the range of 3 to 5) return electrodes located around the stimulating electrode, with a distance of e.g. larger than 2 cm and e.g. less than 10 cm.

According to an embodiment the amplitude of alternating current (ac) may be in the range of 1 mA (milli ampere) to 7 mA:
- Preferable amplitude may be e.g.: 2 mA (e.g. understood as peak-to-peak value).
- Other possible values may be: a value in the range of 1 mA to 5mA,
- Speaking otherwise, the amplitude of the current may be adjusted to result in an electric field in brain stimulation target in the range of 0.08 V/m (Volts per meter) to 0.3 V/m, and/or considering size and shape of the electrode(s), the current density on the skin may be lower than about or than exactly 0.2 mA/cm² (0.2 milli ampere per square centimeters).

An example for the duration of stimulation is 10 min stimulation, 2 min pause, 10 min stimulation, etc. or 5 min stimulation, 2 min pause, 5 min stimulation, etc. The duration of one continuous stimulation phase with tACS may be not more than 20 min.
- In the pilot study 4 by 10 min of stimulation were used, e.g. a total of 40 mins. These are recommendations that result from studies performed and it may be expected that this maximum time will become longer in future, especially with protocols with pauses - like proposed in this documents,
- The stimulations may be adjusted to planned activities during the day. Based on the pilot study, 10 mins (or even shorter) may be efficient for rendering online effects. Based on the pilot study, it is left open what would happen if single stimulation would last longer, without the pauses. In this patent document one focus is on the concept that for prolonged activities pauses length and/or number of pauses is/are adjusted to the expected length of activity to provide support along all the activity, especially automatically. Alternatively, 10 mins stimulation portions or phases may be run on the beginning of activity and then, after the pause, stimulation may be resumed when required (e.g. requested by the patient - "on demand" or triggered automatically based on psychophysiological parameters).

No preparational stimulation (offline, no real time) may be used, i.e. only online stimulation may be used, without performing preparational stimulation.

The time budget and/or the length of the reference time frame may be selected appropriate:
- Based on the pilot study, 40 mins (minutes) of total stimulation per 24 hours may be regarded as safe and efficient, but this may be increased to 60 mins of stimulation or more per 24 hours period in future. This may depend e.g. on results of future studies and/or e.g. on decisions of regulatory authorities (e.g. FDA, Federal Drug Agency for US).

The following considerations may apply to the voltage at the electrode(s):
- Voltage may be in this case a product of current and electrodes impedance. Typical impedance during tACS stimulation may be in the range of 3 kOhms (kilo Ohms) to 10 kOhm or in another appropriate range, which would result in peak voltages between 3 V (Volts) to 10 V (considering e.g. a peak current of 1 mA in a 2 mA peak-to-peak amplitude). With dry or semi-dry electrodes, impedances may be around 20 kOhm, resulting in max voltage of about 20 V. Considering the possibility, that at the beginning of the stimulation the impedance may be slightly higher (e.g. this may be observed in practice - the impedance may drop within some seconds after stimulation starts) the maximum voltage value may be increased to keep current amplitude at the predefined level, e.g. at the start and/or as long as the impedance is increased relative to the value after e.g. 1 minute - the maximum voltage range could be plus/minus 30 V to 40 V. Plus/minus 50 V may e.g. also be possible but such values may be rarely used.

According to an embodiment the number of electrode(s) may depend on the following:
- A second electrode ("counter electrode", "return electrode") may be used to close the circuit. This electrode(s) may be positioned:
- Contralaterally on the head 121 of the user 120/patient - i.e. at least one counter electrode on the right side of the head 121 in case of stimulation electrode positioned on the left side of the head (e.g. over Wernicke/Broca areas),
- Contralaterally and extracephalically - e.g. at least one counter electrode on the right arm, or
- HD-tACS montage arrangement as mentioned above.
In the pilot study extracephalic montage was used (e.g. counter electrode on right arm). However, electrodes located on the right side of the head 121 may be used as well, e.g. comparably large electrodes located over temporal lobe and/or frontal lobe and/or parietal lobe or on mastoid.

A gel may be used between the electrode, especially the active face of the electrode, and the cranium
- However, usage of a gel may depend on the electrode types used. In the intended application, wherein the user performs the stimulation during everyday activities dry electrodes or semi dry electrodes may be used.

Placement of the electrode(s) on the cranium may be as follows:
- Since every stroke patient is different, it may be difficult to find a universal position. One possibility may be to use fMRI (functional Magnetic Resonance Imaging) to identify the target area (e.g. if Broca area or Wernicke area are damaged due to stroke, the target may be located outside their anatomical structures). Alternatively or additionally, computational modelling may be performed to select the position of the electrode(s) that would result in e.g. maximized electric field within the target. In general, most common (but not all possible) positions of electrode following the 10-20 system (commonly used for EEG (Electroencephalography) and brain stimulation) are: C3, C5, FC3, FC5, CP3, CP5, P5, PO7.
- Right-handed patients may be stimulated on the left hemisphere, left-handed patients may be stimulated on the right hemisphere.

Moreover, at least one of the following modifications may be made:
I) The user may decide whether he performs a pre-stimulation or whether he uses real time stimulation.
II) Monitoring or scheduling of a budget may be the common feature of several variants of the invention. This applies to all envisaged support modes explicit by user "on demand", triggered automatically based e.g. on psychophysiological parameters (monitoring), and/or scheduling (indirect monitoring by scheduling), etc.
III) During explicit triggering of stimulation by user/patient it may be checked whether the triggering is performed by the user/patient itself. In the affirmative case, e.g. if yes, stimulation may be allowed. In the negative case, e.g. if not, stimulation may not be allowed. Examples for realization may comprise usage of a finger print sensor, usage of an input ID, etc.
IV) If stimulation is active, it may be checked whether the wearable device is worn. In the affirmative case, e.g. if yes, the budget may be reduced. In the negative case, e.g. if not, the budget may not be reduced even if the electrodes are stimulated but not the brain of user 120/patient. Examples for realization may comprise: checking whether user 120/patient wears or carries the electrodes via resistance on electrode and/or via capacitance, or via other electrical parameters of the electrode(s), etc., usage of separate switch or push button on the headset, e.g. the state of the switch changes if the wearable device is put on the head of user 120/patient, etc.

The overall budget/dose usable within the reference time frame (e.g. within 24 hours) may be a tolerable amount that is tolerable without risks for the health of the user 120/patient. This tolerable amount may be different from capacity of a chargeable or non-chargeable battery of the brain stimulation system 102.

Thus, a mobile, wearable device is proposed that realizes a brain support stimulation application based, e.g. considering a pre-given budget for a reference time frame (e.g. 24 hours). The device may be used advantageous for users/patients suffering from aphasia. However other applications for speech and/or language improving may be envisaged as well.

Although embodiments of the present disclosure and their advantages have been described in detail, it should be understood that various changes, substitutions and alterations can be made therein without departing from the spirit and scope of the disclosure as defined by the appended claims. For example, it will be readily understood by those skilled in the art that many of the features, functions, processes and methods described herein may be varied while remaining within the scope of the present disclosure. Moreover, the scope of the present application is not intended to be limited to the particular embodiments of the system, process, manufacture, method or steps described in the present disclosure. As one of ordinary skill in the art will readily appreciate from the disclosure of the present disclosure systems, processes, manufacture, methods or steps presently existing or to be developed later that perform substantially the same function or achieve substantially the same result as the corresponding embodiments described herein may be utilized according to the present disclosure. Accordingly, the appended claims are intended to include within their scope such systems, processes, methods or steps. The embodiments mentioned in the first part of the description may be combined with each other. The embodiments of the description of figures may also be combined with each other. Further, it is possible to combine embodiments mentioned in the first part of the description with examples of the second part of the description which relates to Figures 1 to 4.

### Reference numerals

- 100: neural (brain) stimulation arrangement
- 102: neural (brain) stimulation system
- 104: volatile memory
- 106: nonvolatile memory
- 108: electronic control unit (ECU)
- 110: user interface (optional)
- 112: connection
- 120: user/patient
- 121: head
- 122: neural (brain) stimulation device
- 124: wireless connection (or wired)
- P1, P1a: program code
- 130: brain
- 132: user condition monitor device
- 134: remote location device
- 200: operation control system (OCS)
- 202: stimulation functionality/unit
- CU: control unit
- 208: user condition monitoring functionality/unit
- 240: session operation protocol interface functionality (optional)
- 242: stimulation planning functionality (optional)
- 244: stimulation dose control functionality (optional)
- 250 to 284: relation
- 289: local input interface
- 300: method
- 302 to 324: method steps
- 402: communication interface unit
- 404: control unit
- 406, E: electrodes
- 408: electrical power unit
- I): supporting mode
- II): training mode

## Claims

1. Neural stimulation system (100) for supporting speech and/or language skills of a user (120), comprising:
- an electrical stimulation unit (202) that is configured to generate an electrical stimulation signal for the brain (130) of the user (120),
wherein the neural stimulation system (100) is configured to support the user (120) by stimulating areas of the brain (130) of the user (120) that are involved in speech and/or language processing and by using stimulation parameters to generate the electrical stimulation signal that are appropriate to enhance the word processing of the user (120).

2. Neural stimulation system (100) according to claim 1, comprising a user interface (110) that is configured to receive input of the user that indicates that the user wants to start stimulation immediately.

3. Neural stimulation system (100) according to claim 1 or 2, comprising a scheduler unit that is configured to provide a planning functionality allowing the user (120) to specify time slots in which he wants to have at least one stimulation,
and wherein preferably the scheduler unit distributes stimulation depending on a stimulation budget to the specified time slots.

4. Neural stimulation system (100) according to any one of the claims 1 to 3, comprising a user condition monitoring unit (208) configured to determine a state of the user (120),
wherein the neural stimulation system (100) is configured to start stimulation or to generate a request to the user (120) in order to start stimulation if an increased arousal and/or engagement or an increased cognitive load is detected based on the determined state of the user (120),
wherein preferably the neural stimulation system (100) is configured to generate a request to the user (120) whether they want to start the stimulation,
and wherein preferably the stimulation is started if the user decides to start stimulation in answer to the request.

5. Neural stimulation system (100) according to any one of the claims 1 to 4, comprising a user condition monitoring unit (208) configured to determine a state of the user (120),
wherein the neural stimulation system (100) is configured to stop stimulation or to generate a request to the user (120) in order to stop stimulation if a decreased arousal and/or engagement or an decreased cognitive load is detected based on the determined state of the user (120),
wherein preferably the neural stimulation system (100) is configured to generate a request to the user (120) whether they want to stop the stimulation,
and wherein preferably the stimulation is stopped if the user (120) decides to stop the stimulation in answer to the request, and/or
wherein the neural stimulation system (100) is configured to stop stimulation if a condition or state of the user (120) is indicative of a pathological state of the user (120).

6. Neural stimulation system (100) according to any one of the claims 1 to 5, wherein the neural stimulation system (100) is configured to support the user (120) by stimulating areas of the brain (130) of the user (120) that are involved in speech and/or language processing and by using stimulation parameters to generate the electrical stimulation signal that are appropriate to enhance the word processing of the user (120) at the same time at which the stimulation is made.

7. The neural stimulation system (102) according to any one of the preceding claims, wherein the user (120) is a patient suffering from an illness that has mitigated his/her speech and/or language processing skills,
preferably suffering from speech and/or language disorders, e.g. from aphasia, and/or wherein the electrical stimulation unit (202) is configured to generate a transcranial alternating current stimulation (tACS),
preferably using a frequency in the range of 60 Hz to 80 Hz or 70 Hz to 80 Hz, 60 Hz to 100 Hz or 70 Hz to 100 Hz, 60 Hz to 150 Hz or 70 Hz to 150 Hz; 60 Hz to 250 Hz or 70 Hz to 250 Hz, preferably at approximate 75 Hz or at exactly 75 Hz.

8. The neural stimulation system (102) according to any one of the preceding claims, comprising a control unit (108, CU) that is configured to control the electrical stimulation unit (202) depending on the stimulation parameters,
wherein preferably the control unit (108, CU) is configured to provide the electrical stimulation signal on demand when the user (120) intends to perform activities requiring intensive speech and language processing and when the user proactively enables brain stimulation device in order to improve speech and/or language performance.

9. The neural stimulation system (102) according to any one of the preceding claims, wherein a control unit (108, CU) is configured to dose the electrical stimulation signal in short intervals during the performed activity, e.g. the neural stimulation system (102) is configured to provide stimulation for a specific period of time, e.g. 10 minutes, and preferably to provide then a pause in the stimulation, preferably for a specific period of time in the range of 2 minutes to 15 minutes or in the range of 6 minutes to 12 minutes, and/or
wherein a control unit (108, CU) or the control unit (108, CU) is configured to control a pause length,
wherein the pause length is preferably a specific period of time, e.g. a constant period of time, and/or a period of time in the range of 1 minute to 5 minutes or in the range of 1 minute to 3 minutes, e.g. 2 minutes, and/or
wherein a control unit (108, CU) or the control unit (108, CU) is configured to allow the user (120) to adjust the pause length, preferably in order to distribute stimulation periods evenly over an anticipated duration of activity of the user (120).

10. The neural stimulation system (102) according to any one of the preceding claims, wherein a control unit (108, CU) is configured to control the duration of stimulation in order not to exceed a maximum allowed total duration during a reference timeframe, wherein the reference timeframe is preferably a 24 hours timeframe,
wherein preferably the duration of stimulation is in the range of 30 minutes to 120 minutes or in the range of 45 minutes to 90 minutes, e.g. 60 minutes per 24 hours, and
wherein preferably a total duration is defined as sum of all stimulation periods performed during the reference time frame, e.g. during the 24 hours timeframe, and/or
wherein the neural stimulation system (102) comprises at least one first stimulation electrode (E) that is configured to be mounted on to the head (121) of the user (120), preferably non-invasively, and
wherein preferably the at least one stimulation electrode (E) is configured to be positioned on the left side of the head (121) of the user (120), e.g. close to areas of the brain (130) related to speech and/or language functions, and/or
wherein the neural stimulation system (102) comprises at least one second electrode (E), e.g. a stimulation electrode or a counter electrode, that is configured to be positioned on the right side of the body of the user or on the right side of the head of the user (120).

11. The neural stimulation system (102) according to any one of the preceding claims, especially according to claim 10, comprising a brain stimulation device (122), preferably comprising a wearable headband,
wherein at least one electrode (E) is attached to the brain stimulation device (122), and wherein preferably the brain stimulation device (122) is configured such that the user can wear it for prolonged periods of time.

12. The neural stimulation system (102) according to any one of the preceding claims, comprising:
- a user condition monitoring unit (208) for detecting a state of the user (120), e.g. mental, physical, etc., and
- a control unit (CU) that is configured to start the stimulation automatically depending on the detected state, e.g. without knowledge of the user (120), or to generate a automatically request to the user (120) in order to start stimulation depending on the detected state, preferably if an increased arousal and/or engagement or an increased cognitive load is detected based on the determined state of the user (120),
wherein the user condition monitoring functionality (208) is configured to determine user condition data indicative for the current condition of the user (120) experienced by the user (120) and/or one or more physiological parameters of the user (120) which may be monitored by the brain stimulation system (102) during performing the speech and language related activities by the user (120),
wherein at least one of the user condition data and/or the one or more physiological parameters is used or are used to trigger the start of the stimulation when the user (120) experiences excessive arousal and/or engagement level or cognitive load in order to support the user (120) in the performed activity,
wherein preferably the user condition data is data indicative for the current arousal and/or engagement level and/or cognitive load of the user (120), e.g. fatigue and/or mental load, and
wherein preferably the one or more physiological parameters of the user (120) are based on electrodermal activity and/or on the pulse frequency and/or heart rate and/or blood pressure of the user (120) and/or on heart rate variability of the heart of the user (120) and/or blood oxygenation level.

13. The neural stimulation system (102) according to any one of the preceding claims, comprising:
- a user condition monitoring unit (208) for detecting a state of the user (120), e.g. mental, physical, etc., and
- a control unit (CU) that is configured to stop the stimulation automatically depending on the detected state, e.g. without knowledge of the user (120), or to generate automatically a request to the user (120) in order to stop the stimulation depending on the detected state, preferably if a decreased arousal and/or engagement or an decreased cognitive load is detected based on the determined state of the user (120) or if a condition or state of the user (120) is indicative of a pathological state of the user (120),
wherein the user condition monitoring functionality (208) is configured to determine user condition data indicative for the current condition of the user (120) experienced by the user (120) and/or one or more physiological parameters of the user (120) which may be monitored by the brain stimulation system (102) during performing the speech and language related activities by the user (120),
wherein at least one of the user condition data and/or the one or more physiological parameters is used or are used to stop the stimulation when the user (120) experiences decreased arousal and/or engagement level or cognitive load in order to stop the support of the user (120) if the user does not need the support stimulation in the performed activity anymore,
wherein preferably the user condition data is data indicative for the current arousal and/or engagement level and/or cognitive load of the user (120), e.g. fatigue and/or mental load, and
wherein preferably the one or more physiological parameters of the user (120) are based on electrodermal activity and/or on the pulse frequency and/or heart rate and/or blood pressure of the user (120) and/or on heart rate variability of the heart of the user (120) and/or blood oxygenation level.

14. The neural stimulation system (102) according to any one of the preceding claims, wherein the neural stimulation system (102) is configured to be portable by the user (120), and/or
wherein the neural stimulation system (102) comprises a user interface configured to allow input of the user (120) and/or to output control information to the user (120).

15. Usage of the neural stimulation system (102) according to any one of the preceding claims,
wherein preferably the word processing of the user (120) comprises hearing of words spoken by at least one other person and understanding the words spoken by the at least one other person and speaking words to the at least one other person, and wherein the neural stimulation system (102) is used to support the user (120) in a working environment of the user (120) or in another environment in which the user (120) has to communicate to the at least one other person with enhanced speech and/or language skills outside of a language training situation.

16. A method for supporting speech and/or language performance of a user (120), comprising:
- Providing a neural stimulation system (102) that is configured to generate an electrical stimulation signal for the brain (130) of the user (120), preferably a neural stimulation system (102) according to any one of the claims 1 to 14,
wherein the neural stimulation system (102) is configured to support the user (120) by stimulating areas of the brain (130) of the user (120) that are involved in speech and/or language processing and by using stimulation parameters to generate the electrical stimulation signal that are appropriate to enhance the word processing of the user at the same time at which the stimulation is made,
wherein preferably the word processing of the user (120) comprises hearing of words spoken by at least one other person and understanding the words spoken by the at least one other person and speaking words to the at least one other person, and
- Supporting the user (120) by the neural stimulation system (102) in a working environment of the user (120) or in another environment in which the user has to communicate to the at least one other person with enhanced speech and/or language skills outside of a language training situation.

17. Neural stimulation device (122), comprising:
a communication interface unit (402),
at least one control unit (404) connected to the communication interface (402),
at least one electrode (E, 406) configured to be controlled by the control unit (404), and
an electrical power unit (408) configured to supply electrical power to the units (402, 404) of the neural stimulation device (122) and to supply power to the at least one electrode (E, 406) in order to perform a brain stimulation of a brain (130) of a user (120) carrying the neural stimulation device (122),
wherein preferably the communication interface (402) is configured to communicate with a neural stimulation system (102), preferably with a neural stimulation system (102) according to any one of the claim 1 to 14, in order to receive and preferably also to confirm digital commands or digital parameters specifying details of an electrical stimulation of a brain of a user who carries the neural stimulation device (122),
wherein preferably the control unit (404) is configured to control a current and/or a voltage applied to the at least one electrode (E, 406) according to the digital commands and/or the digital parameters received via the communication interface (402).

18. A neural stimulation arrangement (100) comprising:
the neural stimulation system (102) according to any one of the claims 1 to 14, and
the neural stimulation device (122) according to claim 17, and
preferably a user condition monitoring device (132) which is configured to monitor one or more physiological parameters of the user (120), preferably physiological parameters indicative for the cognitive workload of the user (120).
